# EUROPEAN PATENT APPLICATION

(11) **EP 4 851 177 A1**
(43) Date of publication of application: **09.12.2026**
(21) Application number: 25747675.4
(22) Date of filing: 22.01.2025
(51) Int. Cl.: C07J 63/00, A61K 31/58, A61K 31/56, A61P 25/00, A61P 9/10, A61P 25/18, A61P 21/00, A61P 25/16, A61P 25/28, A61P 25/08, A61P 25/14, A61P 9/04, A61P 13/12, A61P 29/00

(54) **C-17 CARBONYL-SUBSTITUTED OLEANANE TRITERPENE DERIVATIVE, PREPARATION METHOD THEREFOR, AND USE THEREOF**

(30) Priority: 29.01.2024 CN 202410123583; 09.01.2025 CN 202510036026
(71) Applicant: Yantai University, Yantai, Shandong 264005 (CN)
(72) Inventor: TIAN, Jingwei, Yantai, Shandong 264005 (CN); FU, Fenghua, Yantai, Shandong 264005 (CN); WANG, Hongbo, Yantai, Shandong 264005 (CN); ZOU, Fangxia, Yantai, Shandong 264005 (CN); LI, Chunmei, Yantai, Shandong 264005 (CN)
(74) Representative: Valet Patent Services Limited
(86) International application number: PCT/CN2025/073807
(87) International publication number: WO 2025/162103

(57) **Abstract**

The present invention relates to a C-17 carbonyl-substituted oleanane triterpene derivative, a preparation method therefor, and a use thereof. Provided are a C-17 carbonyl-substituted oleanane triterpene derivative, a use of the compound in the preparation of an NRF2 activator, and preparation of a medicament for treating/preventing diseases. The diseases comprise cerebral small vascular disease, mitochondrial encephalomyopathy, autism spectrum disorder, Rett syndrome, Friedreich ataxia, stroke, hemorrhagic cerebral apoplexy, ischemic cerebral apoplexy, multiple sclerosis, amyotrophic lateral sclerosis, schizophrenia, schizophrenic cognitive impairment, Parkinson's disease, cognitive impairment in Parkinson's disease, Alzheimer's disease, vascular dementia, epilepsy, Huntington's disease, heart failure, myocardial infarction, renal failure, kidney ischemia, etc., or other disease states and conditions which are obvious to a person skilled in the art. Also provided are a prodrug thereof, or a pharmaceutically acceptable salt thereof, or a hydrate or solvate thereof, and a pharmaceutical composition containing same

## Description

### TECHNICAL FIELD

The present application generally relates to the fields of biology and medical technology, and specifically to a C-17 carbonyl-substituted oleanane triterpenoid derivative, a preparation method therefor and a use thereof.

### BACKGROUND ART

Numerous studies have found that oxidative stress may directly or indirectly induce the onset and progression of various diseases, making in-depth research on oxidative stress particularly important. Oxidative stress is caused by severe imbalance between generation of reactive oxygen species (ROS) and reactive nitrogen species (RNS) and the antioxidant defense system, enzymatic and non-enzymatic processes. To combat this damage, the body has developed a complex oxidative stress response system to alleviate cellular damage and to maintain homeostasis by upregulating cytoprotective factors.

Nrf2 belongs to the leucine (CNC) regulatory protein family, comprising Neh1 to Neh6, and is an important transcription factor for the body regulating antioxidant stress response. Keap1 is the specific receptor for Nrf2. Under normal conditions, Nrf2 and Keap1 recognize each other and form a polymer, and the function thereof is inhibited by Keap1. Under conditions such as oxidative stress, Nrf2 is dissociated from Keap1 and activated, initiating the antioxidant response element (ARE), regulating the expression of phase II detoxification enzyme genes and antioxidant enzyme genes, and enhancing cellular resistance to substances such as oxidative stress and electrophilic biomacromolecules. However, current molecules focus solely on inflammatory therapy through Nrf2 agonism, exhibiting poor inhibitory activity and low specificity. Therefore, developing novel Nrf2 activators with both anti-inflammatory and antioxidant effects is of paramount importance.

### SUMMARY OF THE INVENTION

To address the aforementioned technical problems, the present application provides a C-17 carbonyl-substituted oleanane triterpenoid derivative, a preparation method therefor, and a use thereof.

A compound of formula (I) or a pharmaceutically acceptable salt thereof: 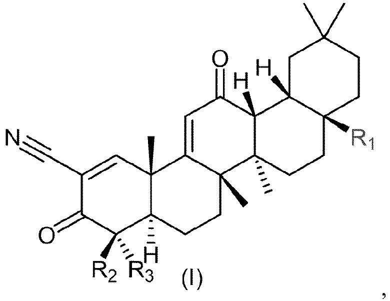
in the above: R₁ is independently selected from the group consisting of: -C(=O)-alkyl, - C(=O)-substituted alkyl, -C(=O)-alkenyl, -C(=O)-substituted alkenyl, -C(=O)-alkynyl, -C(=O)-substituted alkynyl, -C(=O)-heteroaryl, -C(=O)-heteroaryl-R₁', -C(=O)-heteroaryldiyl-R₁'-C(=O)-NH-OH, -C(=O)-N(OH)-alkyl, -C(=O)-N(OH)-substituted alkyl, -C(=O)-N(OH)-alkenyl, - C(=O)-N(OH)-substituted alkenyl, -C(=O)-N(OH)-alkynyl, -C(=O)-N(OH)-substituted alkynyl, - C(=O)-N(OH)-aryl, -C(=O)-N(OH)-aryldiyl-R₁', -C(=O)-N(OH)-heteroaryl, -C(=O)-N(OH)-heteroaryldiyl-R₁', -C(=O)-NH-aryl, -C(=O)-NH-aryldiyl-R₁', -C(=O)-NH-heteroaryl, -C(=O)-NH-heteroaryldiyl-R₁', -C(=O)-O-aryl, -C(=O)-O-aryldiyl-R₁', -C(=O)-O-heteroaryl, -C(=O)-O-heteroaryldiyl-R₁', -C(=O)-CH₂-heteroaryl, -C(=O)-CH₂-heteroaryldiyl-R₁', -C(=O)-CR₂'R₃'-heteroaryl, -C(=O)-CR₂'R₃'-heteroaryldiyl-R₁', -C(=O)-L-amino acid-NH-heteroaryl, and -C(=O)-L-amino acid-NH-heteroaryldiyl-R₁';
and R₂ is hydrogen or methyl; R₃ is hydrogen or methyl.

The compound is further defined as: 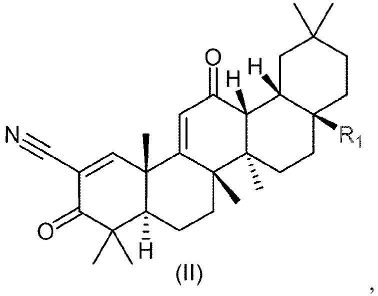
in the above: R₁ is independently selected from the group consisting of: -C(=O)-alkyl, - C(=O)-substituted alkyl, -C(=O)-alkenyl, -C(=O)-substituted alkenyl, -C(=O)-alkynyl, -C(=O)-substituted alkynyl, -C(=O)-heteroaryl, -C(=O)-heteroaryl-R₁', -C(=O)-heteroaryldiyl-R₁'-C(=O)-NH-OH, -C(=O)-N(OH)-alkyl, -C(=O)-N(OH)-substituted alkyl, -C(=O)-N(OH)-alkenyl, - C(=O)-N(OH)-substituted alkenyl, -C(=O)-N(OH)-alkynyl, -C(=O)-N(OH)-substituted alkynyl, - C(=O)-N(OH)-aryl, -C(=O)-N(OH)-aryldiyl-R₁', -C(=O)-N(OH)-heteroaryl, -C(=O)-N(OH)-heteroaryldiyl-R₁', -C(=O)-NH-aryl, -C(=O)-NH-aryldiyl-R₁', -C(=O)-NH-heteroaryl, -C(=O)-NH-heteroaryldiyl-R₁', -C(=O)-O-aryl, -C(=O)-O-aryldiyl-R₁', -C(=O)-O-heteroaryl, -C(=O)-O-heteroaryldiyl-R₁', -C(=O)-CH₂-heteroaryl, -C(=O)-CH₂-heteroaryldiyl-R₁', -C(=O)-CR₂'R₃'-heteroaryl, -C(=O)-CR₂'R₃'-heteroaryldiyl-R₁', -C(=O)-L-amino acid-NH-heteroaryl, and -C(=O)-L-amino acid-NH-heteroaryldiyl-R₁';
and R₂ is methyl; R₃ is methyl.

Preferably, R₁' is independently selected from the group consisting of: -Cl, -F, -Br, -OH, isopropyl, linear/branched alkyl (C≤6), linear/branched alkyl (C≤ 6) substituted with 1-5 halogens, linear/branched alkyl (C≤6) substituted with 1-5 -OH groups, linear/branched alkenyl (C≤6), linear/branched alkenyl (C≤6) substituted with 1-5 halogens, linear/branched alkenyl (C≤6) substituted with 1-5 -OH groups, linear/branched alkynyl (C≤6), linear/branched alkynyl (C≤6) substituted with 1-5 halogens, linear/branched alkynyl (C≤6) substituted with 1-5 -OH groups, 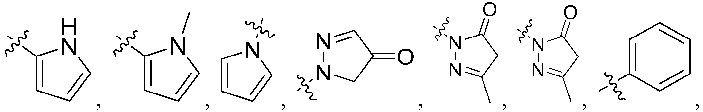 etc.

Preferably, the aryl is selected from the group consisting of: 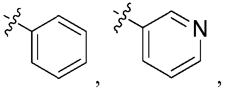 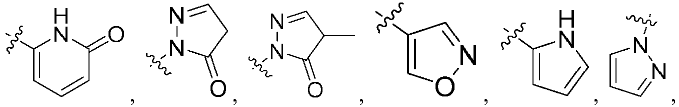 etc.

Preferably, the substituted alkyls, substituted alkenyls, substituted alkynyls, alkyls, alkenyls, and alkynyls have a C chain length of ≤6 and can be linear, branched, or cyclic. The substituents are 1-5 -SO₃H, -OH, -F, -Br, -Cl, methyl, ethyl and/or propyl groups.

Further preferably, the compound, pharmaceutically acceptable salt, or stereoisomer is as follows:

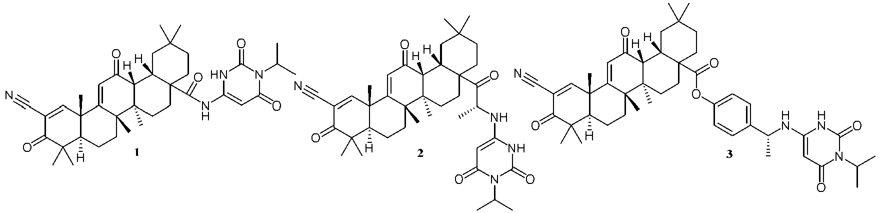

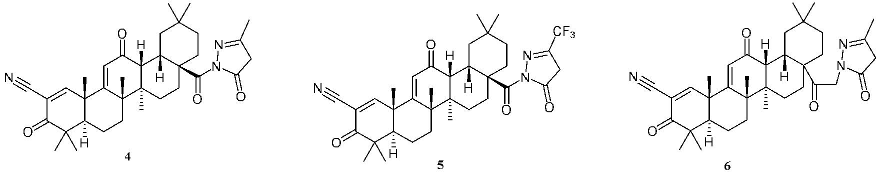

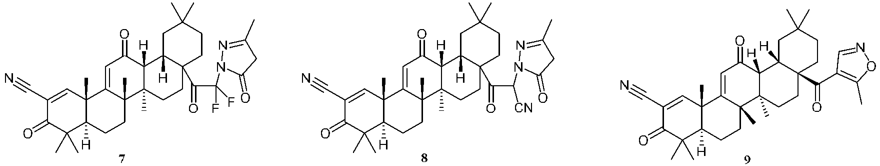

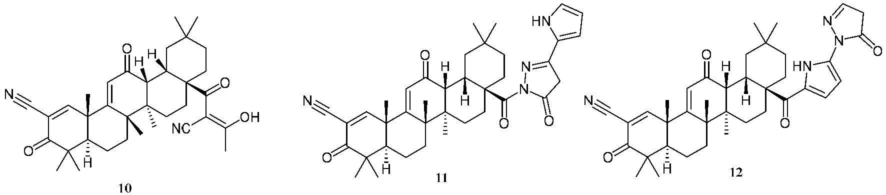

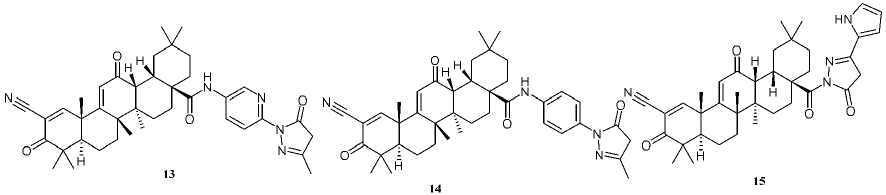

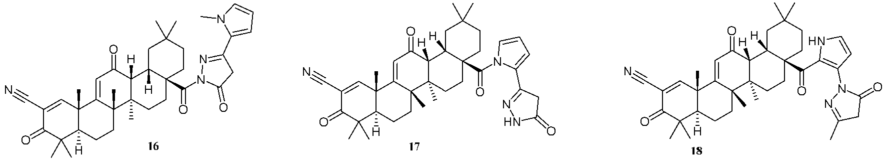

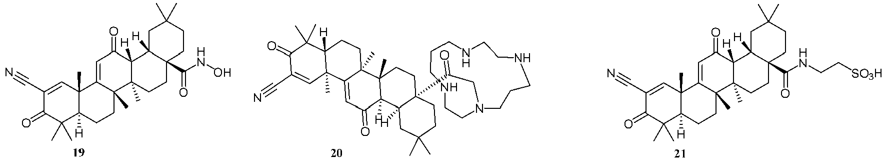

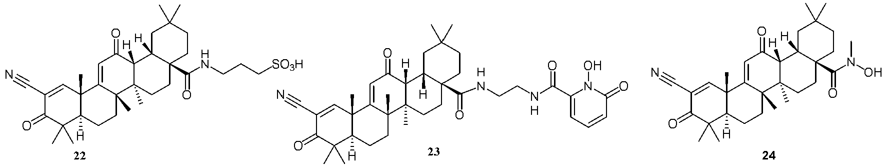

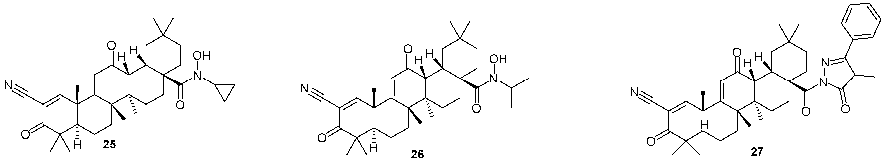

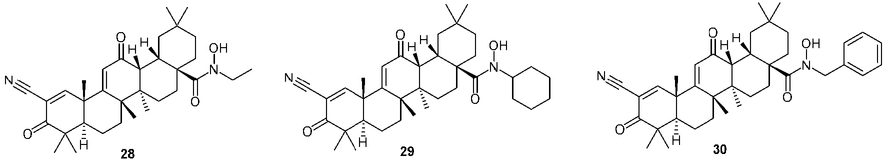

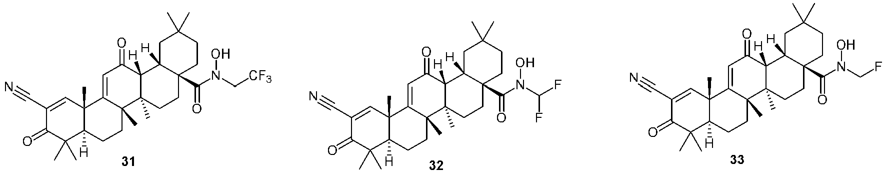

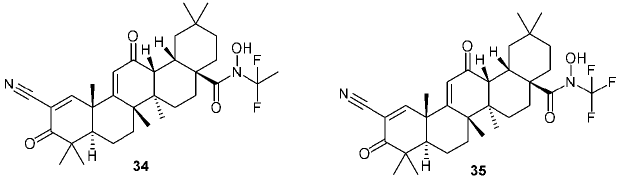

The present application further provides a use of the above-mentioned compound, pharmaceutically acceptable salt, stereoisomer thereof in the preparation of an NRF2 activator.

Biological experiment of the present application has shown that: several compounds in the above compounds of the present application all exhibit single-digit nanomolar human Nrf2 receptor agonist activity, significantly better than the effect of the existing NRF2 activator control drug Omaveloxolone. Furthermore, the experiment further demonstrates that the compounds of the present application possess activities such as scavenging DPPH free radicals, inhibiting formation of lipid peroxide MDA, and intervening in ferroptosis, to exert antioxidant effect.

The present application further provides the use of the above-mentioned compound, its pharmaceutically acceptable salt, stereoisomer, or pharmaceutical composition in the preparation of a medicament for treating and/or preventing a disease in a patient, and the use of the above-mentioned compound, or its pharmaceutically acceptable salt or stereoisomer, or pharmaceutical composition in the preparation of medicaments. Furthermore, the prepared medicament is used for the prevention or treatment of the disease including cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis (ALS), schizophrenia, schizophrenia with cognitive impairment, Parkinson's disease, Parkinson's cognitive impairment, Alzheimer's disease, vascular dementia, epilepsy, Huntington's chorea, heart failure, myocardial infarction, renal failure, and renal ischemia, etc. According to valid experimental data, the medicament prepared from the above-mentioned compound, its pharmaceutically acceptable salt, or stereoisomer of the present application exhibits significant therapeutic effects, particularly effective for stroke, multiple sclerosis, and amyotrophic lateral sclerosis.

Preferably, the use of the compound, its pharmaceutically acceptable salt, or stereoisomer in the preparation of a medicament for the prevention or treatment of stroke, multiple sclerosis, and amyotrophic lateral sclerosis.

Beneficial Effects: the present application, for the first time, synthesizes the compound represented by formula (I) or a pharmaceutically acceptable salt thereof, namely a C-17 carbonyl-substituted oleanane triterpenoid derivative. Furthermore, the present application provides a method for preparing the compound represented by formula (I) through specific examples. Moreover, the present application provides the use of the compound represented by formula (I) or a pharmaceutically acceptable salt thereof in the preparation of an NRF2 activator. The C-17 carbonyl-substituted oleanane triterpenoid derivative disclosed in the present application, while maintaining the strong Nrf2 agonist effect, can also exert antioxidant effects through mechanisms such as scavenging DPPH free radicals, inhibiting the generation of lipid peroxide MDA, or inhibiting ferroptosis. Finally, the present application further provides a pharmaceutical composition including any one of the above-mentioned compounds or pharmaceutically acceptable salts thereof and a pharmaceutically acceptable carrier. The pharmaceutical composition may be prepared into various pharmaceutically acceptable dosage forms, such as tablet, capsule, oral liquid, granule, injection, or various sustained-release or controlled release preparations. The pharmaceutical composition may be administered orally or parenterally (e.g., intravenously, subcutaneously, or locally). The administration dosage may be appropriately adjusted according to the patient's age, gender, and disease type. Diseases that may be prevented or treated in the future include cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, schizophrenia with cognitive impairment, Parkinson's disease, Parkinson's cognitive impairment, Alzheimer's disease, vascular dementia, epilepsy, Huntington's chorea, heart failure, myocardial infarction, renal failure, and renal ischemia, etc. The drugs are significantly effective in treating stroke, multiple sclerosis, and amyotrophic lateral sclerosis.

### BRIEF DESCRIPTION OF THE DRAWINGS

FIG. 1 shows effects of compounds 19 and 28 on mNSS score in rats with ischemic stroke, where compared with the sham group: ###p < 0.001; and compared with the model group: ***p < 0.001.
FIG. 2 shows effects of compounds 19 and 28 on the infarct size in rats with ischemic stroke, where compared with the sham group: ###p < 0.001; and compared with the model group: ***p < 0.001.
FIG. 3 shows effect of compound 24 on neurological function score in EAE model mice, where compared with the sham group: ***p < 0.001; compared with the model group: #p < 0.05, ##p < 0.01, ###p < 0.001; and compared with the Omaveloxolone 15 mg/kg group: ^{&&&}p < 0.001.
FIG. 4 shows effect of compound 24 on body weight in EAE model mice, where compared with the sham group: ***p < 0.001; compared with the model group: #p < 0.05, ###p < 0.001; and compared with the Omaveloxolone 15 mg/kg group: ^{&&&} p < 0.001.
FIG. 5 shows effects of compounds 26 and 31 on the onset time in SOD1 G93A mice, where compared with the control group: ***p < 0.001; compared with the model group: ^{#}p< 0.05, ^{##}p < 0.01; and compared with the Omaveloxolone 3 mg/kg group: ^{&}p < 0.05.
FIG. 6 shows effects of compounds 26 and 31 on motor coordination ability in SOD1 G93A mice, where compared with the control group: *p < 0.05, ***p < 0.001; compared with the model group: ^{###}p < 0.001; and compared with the Omaveloxolone 3 mg/kg group: ^{&}p < 0.05.
FIG. 7 shows effect of compounds 26 and 31 on muscle endurance in SOD1 G93A mice, where compared with the control group: *p < 0.05, ***p < 0.001; compared with the model group: ^{###}p < 0.001; and compared with the Omaveloxolone 3 mg/kg group: ^{&}p < 0.05.

### DETAILED DESCRIPTION OF PREFERRED EMBODIMENTS

The present application will be further illustrated below with reference to specific examples and experimental examples, but this does not limit the scope of the application in any way.

The compound structure of the present application was determined by nuclear magnetic resonance (NMR) and/or liquid chromatography-mass spectrometry (LC-MS).

NMR chemical shifts (δ) were given in parts per million (ppm). NMR was determined by using an AVANCE III600 NMR spectrometer with deuterated dimethyl sulfoxide (DMSO-d₆), deuterated methanol (CD₃OD), and deuterated chloroform (CDCl₃) as determination solvents, and tetramethylsilane (TMS) as the internal standard.

Liquid chromatography-mass spectrometry (LC-MS) was performed on a Shimadzu LCMS2020 mass spectrometer (Japan). HPLC was performed using a Shimadzu LC20A liquid chromatograph (Japan).

The silica gel plates used for thin-layer chromatography were silica gel plates from Yantai Jiangyou, with the specification of 0.2 mm ± 0.03 mm for TLC, and the specification of 0.4 mm-0.5 mm for thin-layer chromatography for product isolation and purification.

Unless otherwise specified in the examples, the SFC separation conditions are as follows: column model: DAICEL CHIRALPAK IC (250mm*30mm, 10um); mobile phase: [CO2-i-PrOH/ACN]; B%: 40%, isocratic elution mode.

In the present application, if specific experimental conditions were not specified, conventional experimental conditions or conditions recommended by the manufacturer shall be followed. If the manufacturer of the reagents or instruments used was not specified, conventional products could all be purchased commercially.

In the present application, the experimental results were expressed as average values.

The detection indicators in the present application were: human Nrf2 receptor function test (agonist detection); DPPH (2,2-diphenyl-1-picrylhydrazyl) free radical scavenging capacity test; MDA (malondialdehyde) anti-lipid peroxidation capacity test.

### Example 1

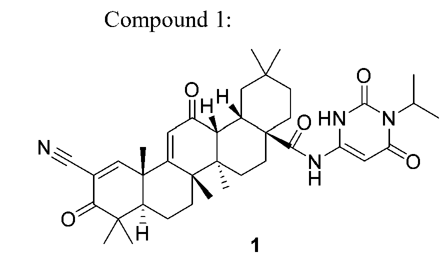

Synthesis route:

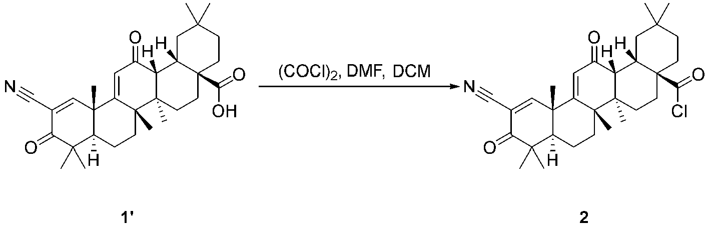

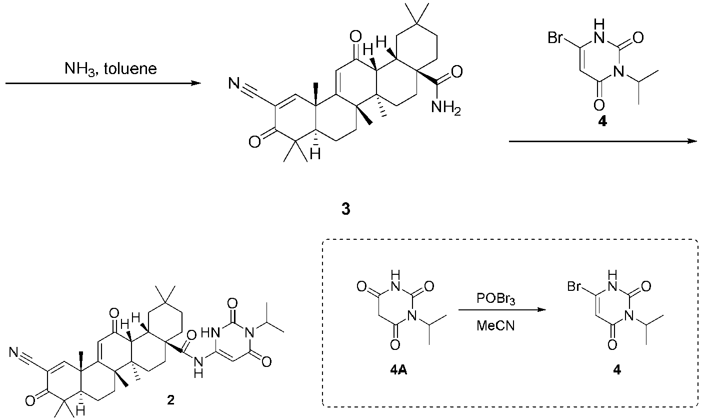

### Synthesis of intermediate 2'

The raw material **1'** (300 mg, 610.18 µmol) was added and dissolved in anhydrous dichloromethane (3 mL), followed by adding N,N-dimethylformamide (22.30 mg, 309.09 µmol) and oxaloyl chloride (309.79 mg, 2.44 mmol, 213.65 µL), and continuing to stir at 25°C for 1 h. After LCMS detected that the reaction was completed, the reaction solution was concentrated to obtain the intermediate **2'** (300 mg, crude product, white solid). LCMS (methyl ester): rt=0.660 min, 506.3 [M+H]⁺.

### Step 2: Synthesis of intermediate 3

Ammonia gas (30.05 mg, 1.76 mmol) was introduced into toluene (4 mL) at -70°C for 10 min. Then, the intermediate **2'** (0.3 g, 588.11 µmol) was added, followed by stirring at -70°C for 1 h. After LCMS detected that the reaction was completed, the reaction mixture was concentrated under reduced pressure to obtain a crude product. The crude product was purified by reversed-phase column chromatography (C₁₈ column, 0.1% formic acid aqueous solution) and lyophilized to obtain an intermediate **6** (250 mg, yield 86.6%, yellow solid). LCMS: rt=0.557 min, 491.3 [M+H]⁺; purity: 100%.

### Step 3: Synthesis of intermediate 4

The intermediate **4A** (5 g, 29.38 mmol) was dissolved in anhydrous acetonitrile (50 mL), and phosphorus tribromooxy (12.64 g, 44.07 mmol) was added under nitrogen atmosphere. The reaction solution was stirred at 80°C for 0.5 h. After the reaction was completed, the reaction mixture was added and quenched in room-temperature water (50 mL) at room temperature, stirred for 1 h, and then filtered and concentrated to obtain a crude product, which was purified by column chromatography (silica gel column, petroleum ether/ethyl acetate=2/1~1/1) to give the intermediate **4** (3.3 g, yield 45.92%, yellow solid). LCMS: rt=0.381, 0.749 min, 233.0 /235.0 [M+H]⁺; purity 95.34% ¹H NMR (400 MHz, DMSO-*d6*) δ=5.93 (s, 1H), 5.00-4.86 (m, 1H), 1.33 (d, *J*=7.0 Hz, 6H).

### Step 4: Synthesis of compound 1

The intermediate **3** (50 mg, 101.90 µmol) and intermediate **4** (35.62 mg, 152.85 µmol) were dissolved in dioxane (4 mL), and after purging with nitrogen three times, PEPPSI Pd (5.99 mg, 7.13 µmol) and sodium tert-butoxide (29.38 mg, 305.70 µmol) were added. After purging with nitrogen three times, the mixture was stirred at 100°C for 2 h. After LCMS detected that the reaction was completed, the reaction mixture was filtered under reduced pressure and concentrated to obtain a crude product. The crude product was purified by preparative chromatography (C₁₈ column, 0.1% formic acid solution) and lyophilized to give the compound **1** (4.2 mg, white solid, yield 4.06%). LCMS: rt=1.560 min, 643.4 [M+H]⁺; purity 94.25%; ¹H NMR (400 MHz, DMSO-*d6)* δ=10.81-10.41 (m, 1H), 9.65-9.28 (m, 1H), 8.65 (s, 1H), 6.22 (s, 1H), 6.03 (br s, 1H), 5.04-4.92 (m, 1H), 3.05 (br d, *J*=4.2 Hz, 1H), 2.84 (br d, *J*=13.6 Hz, 1H), 2.08-1.98 (m, 1H), 1.87-1.73 (m, 3H), 1.67-1.57 (m, 4H), 1.44-1.40 (m, 4H), 1.34 (d, J=7.0 Hz, 6H), 1.28-1.22 (m, 6H), 1.19-1.12 (m, 5H), 1.08-1.00 (m, 4H), 0.96 (s, 3H), 0.89 (br d, *J*=11.6 Hz, 6H).

### Example 2

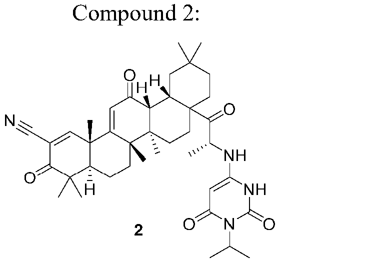

Synthesis route:

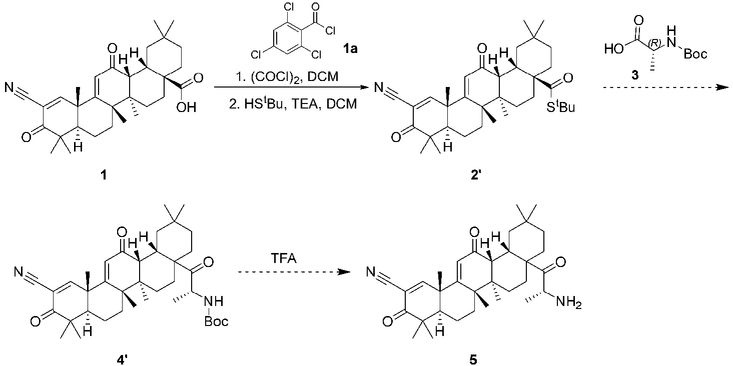

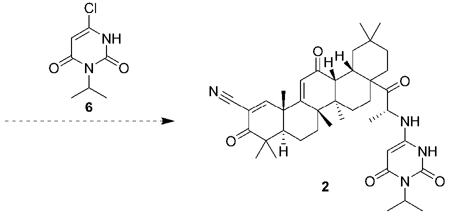

### Example 3

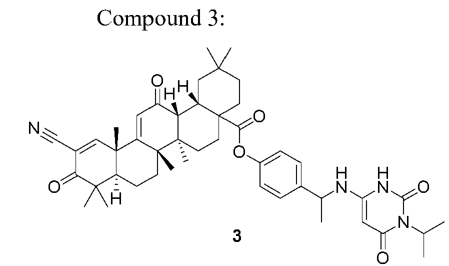

Synthesis route:

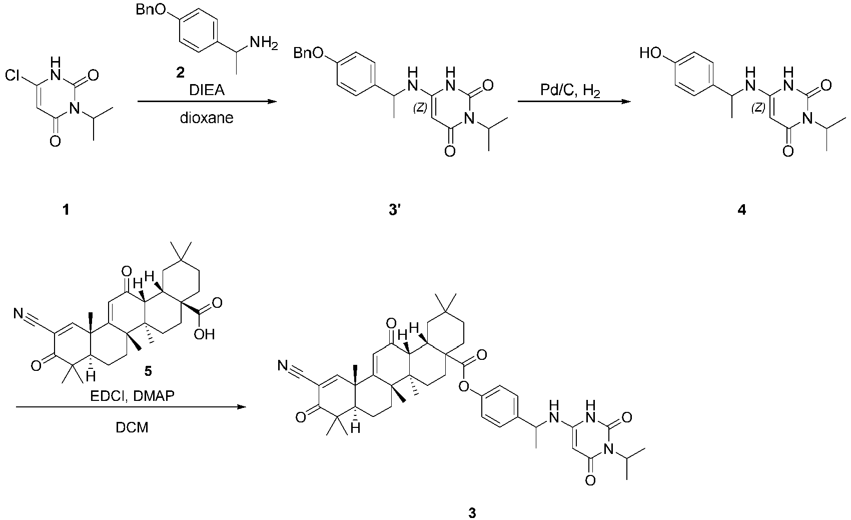

### Step 1: Synthesis of intermediate 3'

The raw material **1** (1 g, 5.30 mmol) and raw material **2** (1.57 g, 6.89 mmol) were added and dissolved in dioxane (10 mL), followed by adding diisopropylethylamine (2.06 g, 15.91 mmol, 2.77 mL), and undergoing microwave reaction at 120°C for 3 h. After LCMS detected that the reaction was completed, the reaction solution was concentrated to obtain a crude product. The crude product was purified by column chromatography (silica gel column, petroleum ether/ethyl acetate=1/0~dichloromethane/anhydrous methanol=1/1) to obtain a crude product. The crude product was slurried with methanol (10 mL) and filtered to obtain the intermediate **3'** (0.9 g, yield 45.95%, gray solid). LCMS: rt=0.506 min, 380.1 [M+H]⁺; purity 96.28%; ¹H NMR (400 MHz, DMSO-*d₆*) δ=9.75 (br s, 1H), 7.46-7.30 (m, 5H), 7.25 (d, *J*=8.6 Hz, 2H), 6.99 (d, *J*=8.8 Hz, 2H), 6.44 (br d, *J*=6.6 Hz, 1H), 5.08 (s, 2H), 4.96-4.84 (m, 1H), 4.49-4.38 (m, 1H), 4.34 (s, 1H), 1.36 (d, *J*=6.8 Hz, 3H), 1.29-1.23 (m, 6H).

### Step 2: Synthesis of intermediate 4

The intermediate **3'** (450 mg, 1.19 mmol) was added and dissolved in anhydrous methanol (200 mL), followed by adding wet palladium on carbon (0.5 g, 10%) under nitrogen protection, purging with hydrogen three times, and then stirring at 25°C for 2 h. After LCMS detected that the reaction was completed, the reaction solution was filtered and concentrated to obtain a crude product. The crude product was purified by reversed-phase column chromatography (C₁₈ column, 0.1% formic acid aqueous solution), and extracted to give the intermediate **4** (120 mg, yield 34.27%, yellow solid). LCMS: rt=0.397 min, 290.1 [M+H]⁺, purity: 98.23%.

### Step 3: Synthesis of compound 3

The intermediate **5** (50 mg, 101.70 µmol) and intermediate **4** (35.31 mg, 122.04 µmol) were added and dissolved in dichloromethane (0.5 mL), followed by adding EDCI ((1-ethyl-3-(3-dimethylaminopropyl)carbodiimide hydrochloride) (23.39 mg, 122.04 µmol) and 4-dimethylaminopyridine (12.42 mg, 101.70 µmol), stirring at 25°C for 12 h, and then stirring at 40°C for 2 h. After LCMS detected that the reaction was completed, the reaction mixture was filtered under reduced pressure and concentrated to obtain a crude product. The crude product was purified by preparative chromatography (C₁₈ column, 0.1% formic acid solution), and lyophilized to give compound **3** (28.6 mg, white solid, yield 36.02%). LCMS: rt=1.746 min, 763.4 [M+H]⁺; purity 97.73%; ¹H NMR (400 MHz, DMSO-d6) δ=9.81 (s, 1H), 8.67 (s, 1H), 7.39 (d, *J*=8.4 Hz, 2H), 7.07 (d, *J*=8.4 Hz, 2H), 6.54 (br d, *J*=6.6 Hz, 1H), 6.26 (s, 1H), 4.98-4.84 (m, 1H), 4.53 (br t, *J*=6.6 Hz, 1H), 4.34 (d, *J*=2.0 Hz, 1H), 2.94 (br d, *J*=3.4 Hz, 2H), 2.08-1.93 (m, 2H), 1.89-1.64 (m, 7H), 1.52-1.43 (m, 6H), 1.39 (br d, *J*=6.8 Hz, 3H), 1.36-1.30 (m, 4H), 1.27 (br d, *J*=6.8 Hz, 8H), 1.18 (s, 3H), 1.07 (s, 3H), 0.99 (s, 3H), 0.95 (s, 3H), 0.91 (s, 3H).

### Example 4

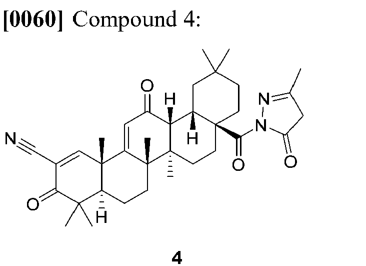

Synthesis route 1:

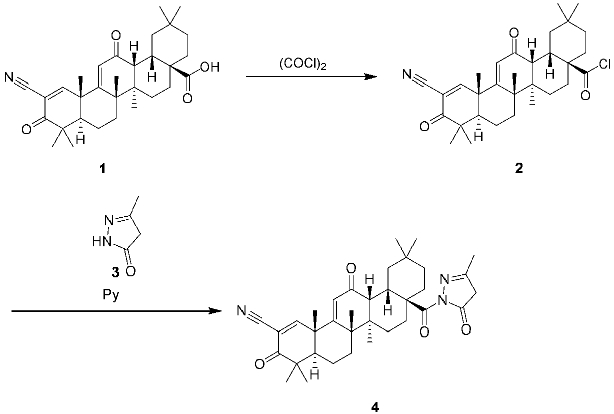

### Step 1: Synthesis of intermediate 2

The raw material 1 (500 mg) was dissolved in anhydrous dichloromethane (5 mL), followed by adding N,N-dimethylformamide (7.82 µL), then purging with a nitrogen balloon, then slowly adding oxaloyl chloride (356.09 µL) to a reaction flask, reacting at 25 °C for 1 h. After LC-MS detected that the reaction was completed, the reaction solution was concentrated under reduced pressure to obtain the residue. The obtained product was used directly in the next reaction without purification. The intermediate 2 (555 mg, crude product) was faint yellow solid powder.

### Step 2: Synthesis of compound 4

The material 3 (5-methyl-2,4-dihydro-3H-pyrazol-3-one, 46.16 mg) was dissolved in pyridine (1 mL) and cooled to 0°C in an ice-water bath under nitrogen protection. The intermediate 2 (160 mg) was dissolved in pyridine (1 mL) and then slowly added to the reaction flask. The reaction was carried out in an ice-water bath for 5 h. After LCMS detected that the raw material reacted completely, the reaction solution was poured into water (3 mL), extracted with ethyl acetate (2 mL*3) and separated, and the organic phase was washed with saturated brine (2.5 mL*2) and then dried over anhydrous sodium sulfate and concentrated. The crude product was isolated by preparative chromatography (C₁₈ column, 0.1% formic acid solution) and lyophilized to obtain the compound **4** (27 mg, yield 14.45%). LCMS: Rt=0.631 min, 572.5 [M+H]⁺; HPLC: Rt=2.098 min; ¹H NMR (400 MHz, CHLOROFORM-d) δ=8.05 (s, 1H), 5.98 (s, 1H), 5.92 (s, 1H), 3.13 (br s, 2H), 2.36-2.32 (m, 3H), 2.07-1.99 (m, 3H), 1.79 (br s, 11H), 1.49 (s, 3H), 1.38 (s, 3H), 1.27 (s, 5H), 1.18 (s, 3H), 1.05 (d, J=4.0 Hz, 6H), 0.94 (s, 3H) .

### Synthesis route 2:

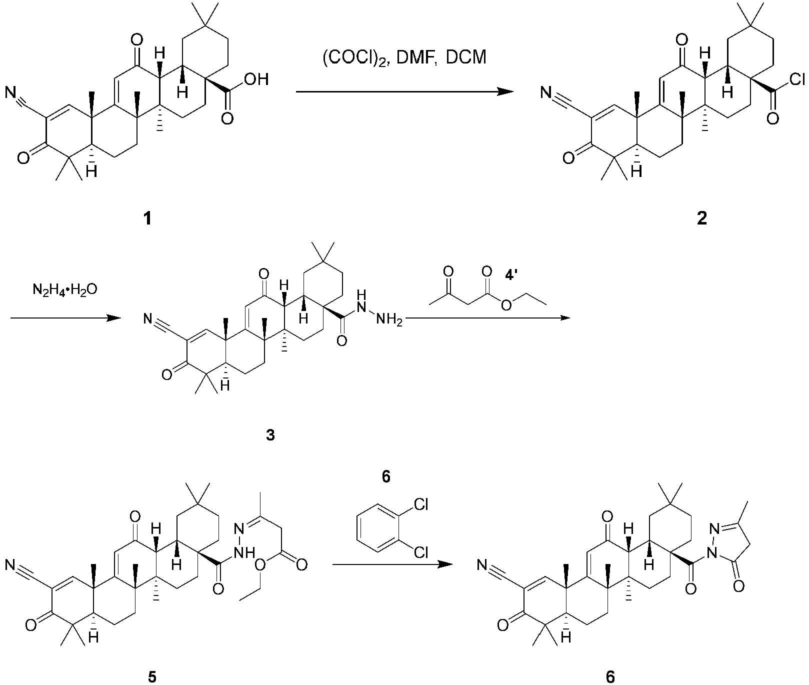

### Step 1: Synthesis of intermediate 2

The raw material 1 (1 g) was dissolved in anhydrous dichloromethane (10 mL), followed by adding N,N-dimethylformamide (15.65 µL), then purging by using a nitrogen balloon, subsequently slowly adding oxaloyl chloride (712.16 µL) to the reaction flask, and reacting at 25°C for 1 h. After LCMS detected that the raw material reacted completely (quenched with methanol), the reaction solution was concentrated under reduced pressure to obtain a solid. The obtained product was used directly in the next step reaction without purification. The intermediate **2** (1.1 g, crude product) was faint yellow solid powder.

### Step 2: Synthesis of intermediate 3

Hydrazine hydrate (1.05 Ml, 98%) was dissolved in anhydrous dichloromethane (5 mL), and cooled to 0°C in an ice-water bath under nitrogen protection. The intermediate **2** (1.1 g) was dissolved in anhydrous dichloromethane (5 mL) and then slowly added to the reaction flask. The reaction was carried out in an ice-water bath for 1 h. After LCMS detected that the raw material reacted completely, the reaction solution was concentrated. The crude product was isolated by preparative chromatography (C₁₈ column, 0.1% formic acid solution), and extracted with ethyl acetate (20 mL*2) after acetonitrile eluate was concentrated. The organic phase was washed with saturated brine (30 mL), then dried over anhydrous sodium sulfate, and concentrated to give compound **3** (580 mg, 1.15 mmol, yield 53.19%). **LCMS:** rt=0.502 min, 506.3 [M+H]⁺.

### Step 3: Synthesis of intermediate 5

The intermediate 3 (580 mg) and material 4' (145.20 µL) were dissolved in anhydrous ethanol (7 mL), followed by adding acetic acid (6.57 µL) and reacting at 50 °C for 3 h. LCMS monitoring showed 40% of the raw material remained and 35% of the product was produced. The reaction solution was concentrated under reduced pressure, rotary evaporated to dryness, and directly used in the next step. Crude product 5 (600 mg, yield 84.67%) was a brown solid. **LCMS:** rt=0.599 min, 618.5 [M+H]⁺.

### Step 4: Synthesis of compound 4

The intermediate compound **5** (500 mg) was dissolved in material 6 (1,2-dichlorobenzene, 10 mL), and heated to 145°C for reacting for 12 h. LCMS monitoring showed that the raw material was consumed completely and a product was formed. The reaction solution was concentrated under reduced pressure, stirred with silica gel, and isolated by normal-phase column chromatography (petroleum ether:ethyl acetate=0/0-3/1) to obtain the crude product, which was isolated and purified by preparative chromatography (C₁₈ column, 0.1% formic acid solution) to give the compound **4** (39.9 mg, 69.30 µmol, yield 8.56%). LCMS: rt=1.655 min, 572.4 [M+H]⁺; HPLC: rt=2.223 min; ¹H NMR (400 MHz, CHLOROFORM-d) δ=8.05 (s, 1H), 5.98 (s, 1H), 5.88 (s, 1H), 3.14 (br d, *J*=4.0 Hz, 2H), 2.33 (s, 3H), 2.02 (s, 3H), 1.95-1.80 (m, 3H), 1.75-1.54 (m, 4H), 1.49 (s, 3H), 1.43-1.33 (m, 5H), 1.31-1.23 (m, 6H), 1.17 (s, 3H), 1.05 (d, *J*=4.4 Hz, 6H), 0.94 (s, 3H) .

### Example 5

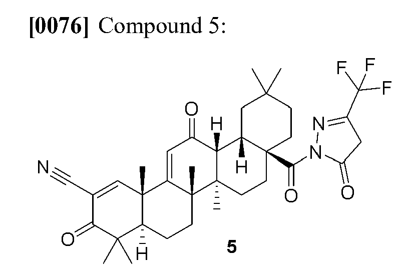

Synthesis route:

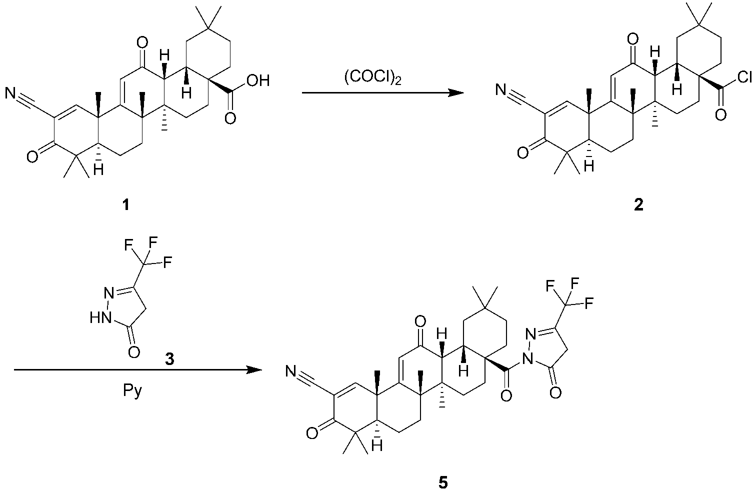

### Step 1: Synthesis of intermediate 2

The raw material 1 (500 mg, 1.02 mmol, 1 eq) was dissolved in anhydrous dichloromethane (5 mL), followed by adding N,N-dimethylformamide (7.43 mg, 101.70 µmol, 7.82 µL, 0.1 eq) under nitrogen protection, subsequentially slowly adding oxaloyl chloride (516.33 mg, 4.07 mmol, 356.09 µL, 4 eq) to the reaction flask, and reacting at 25°C for 1 h. After LCMS detected that the raw material reacted completely, the reaction solution was concentrated under reduced pressure to obtain the residue. The obtained product was used directly in the next step reaction without purification. The compound **2** (555 mg, crude product) was faint yellow solid powder.

### Step 2: Synthesis of compound 5

The material **3** (66.78 mg, 439.12 µmol, 0.8 eq) was dissolved in pyridine (2 mL), purged with a nitrogen balloon, and then cooled to 0°C in an ice-water bath. The intermediate 2 (280 mg, 548.91 µmol, 1 eq) was dissolved in pyridine (2 mL) and then slowly added to the reaction flask, for reaction in an ice-water bath for 5 h. After LCMS detected that the raw material reacted completely, the reaction solution was poured into water (5 mL), extracted with ethyl acetate (5 mL*3) and separated, and the organic phase was washed with saturated brine (5 mL*2) and then dried over anhydrous sodium sulfate and concentrated. The crude product was isolated by preparative chromatography (C₁₈ column, 0.1% formic acid solution) and then lyophilized to obtain the compound **5** (80 mg, 122.10 µmol, yield 22.24%). LCMS: Rt=1.802 min, 626.4 [M+H]⁺; HPLC: Rt=2.388 min ; ¹H NMR (400 MHz, DMSO-*d6*) δ=14.30-13.93 (m, 1H), 8.67 (s, 1H), 6.65 (br s, 1H), 6.26 (s, 1H), 2.91 (br s, 2H), 2.09-1.84 (m, 4H), 1.72-1.61 (m, 5H), 1.50-1.43 (m, 6H), 1.30-1.22 (m, 6H), 1.18 (s, 3H), 1.07 (s, 3H), 0.98 (s, 3H), 0.94 (s, 3H), 0.91 (s, 3H) .

### Example 6

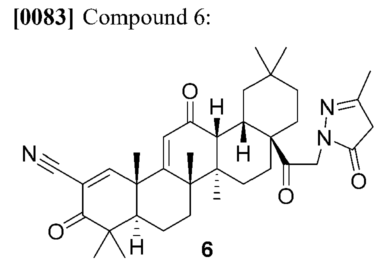

### Synthesis route:

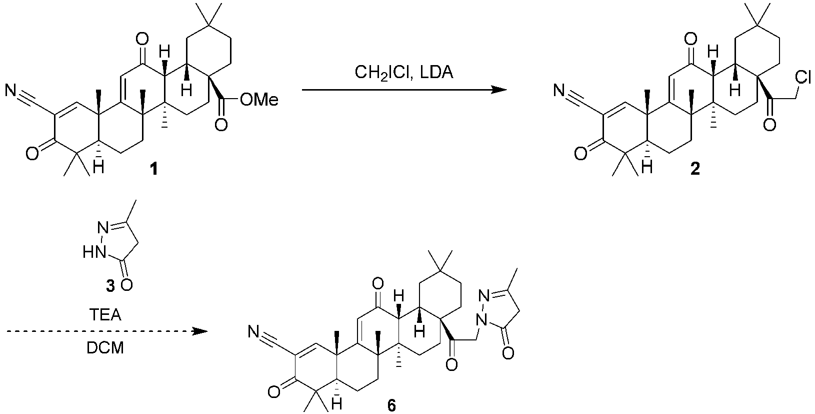

### Example 7

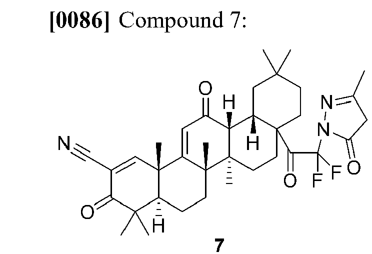

### Synthesis route:

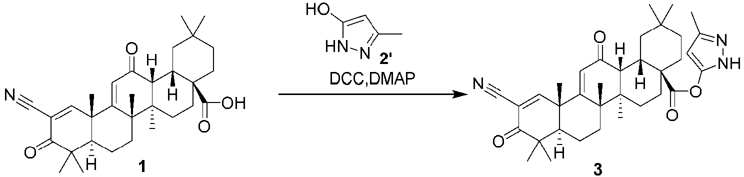

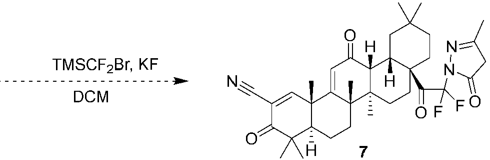

### Example 8

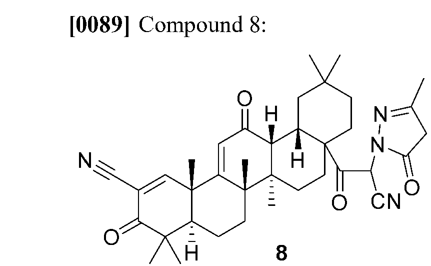

### Example 9

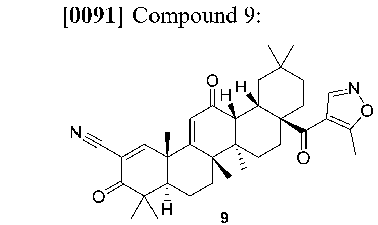

### Synthesis route:

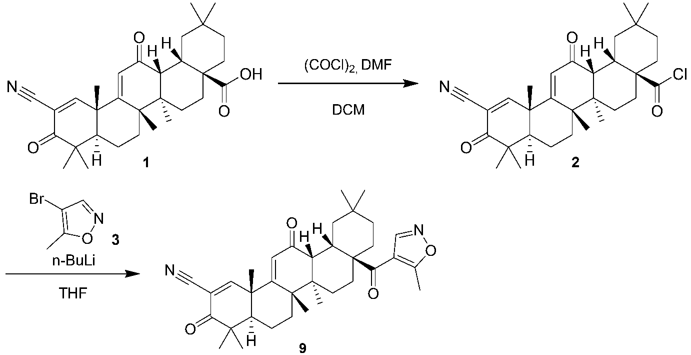

### Example 10

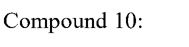

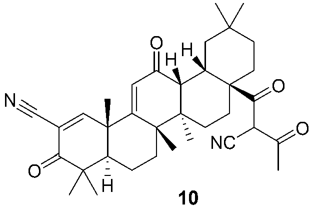

Synthesis route:

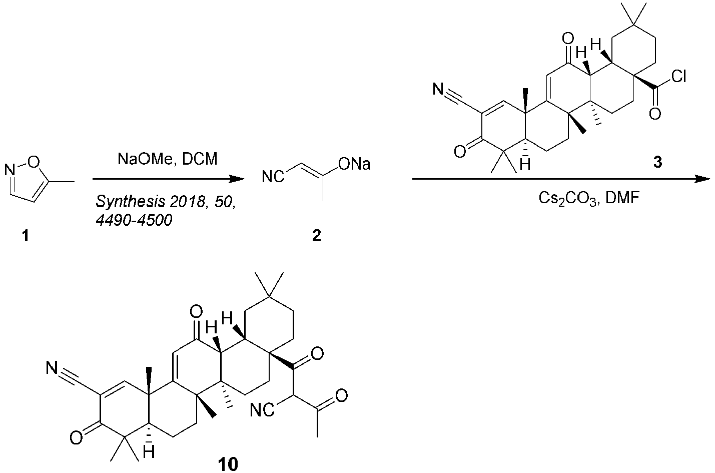

### Example 11

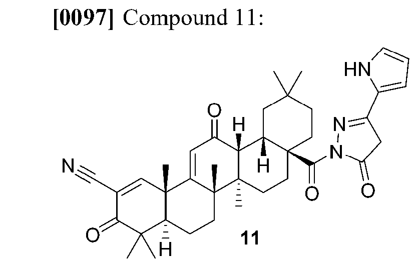

### Synthesis route:

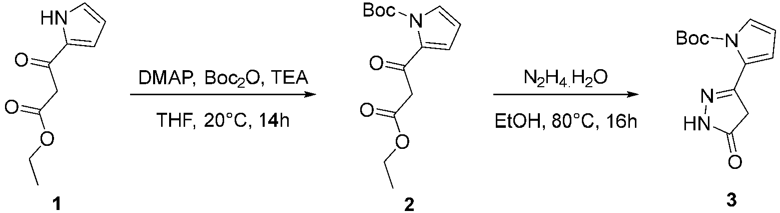

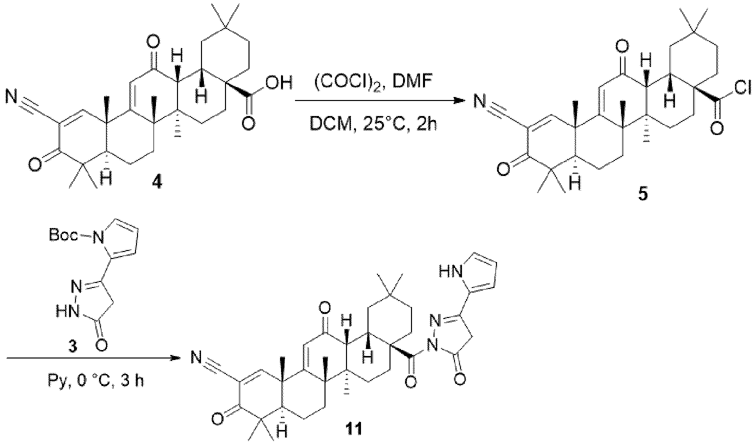

### Step 1: Synthesis of intermediate 2

The compound 1 (1 g, 5.52 mmol, 1 eq), triethylamine (614.32 mg, 6.07 mmol, 845.01 µL, 1.1 eq), Boc2O (1.32 g, 6.07 mmol, 1.39 mL, 1.1 eq), and DMAP (67.43 mg, 551.91 µmol, 0.1 eq) were added to anhydrous tetrahydrofuran (20 mL). The reaction solution was stirred at 20°C for 14 h. LCMS showed that the reaction was completed. The reaction solution was concentrated under reduced pressure to obtain a crude product. The crude product was purified by column chromatography (silica, petroleum ether:ethyl acetate=1:0~4:1) to give the intermediate 2 (1.1 g, 3.44 mmol, yield 62.35%, purity 88%) which was yellow oily. LCMS: rt=0.524 min, 304.1 [M+Na]+, purity: 87.557%.

### Step 2: Synthesis of intermediate 3

The compound 2 (1.1 g, 3.91 mmol, 1 eq) and hydrazine hydrate (4.27 g, 83.59 mmol, 4.14 mL, purity 98%, 21.38 eq) were added to anhydrous ethanol (15 mL). The reaction solution was heated at 80°C for 16 h under a nitrogen atmosphere, and LCMS showed that the reaction was completed. After cooling to room temperature, the reaction solution was concentrated under reduced pressure to give the product (900 mg, 3.61 mmol, yield 92.33%, purity 95%) which was yellow oily. LCMS: rt=0.303 min, 150.0 [M-C5H9O2+H], purity: 94.705%. ¹H NMR (400 MHz, DMSO-d6) δ=11.21 (s, 1H), 6.70 (s, 1H), 6.23 (br d, J=2.0 Hz, 1H), 6.00 (t, J=2.8 Hz, 1H), 3.41 (br s, 2H), 1.36 (s, 9H).

### Step 3: Synthesis of intermediate 5

The compound 4 (100 mg, 203.39 µmol, 1 eq), DMF (dimethylformamide, 7.43 mg, 101.70 µmol, 7.82 µL, 0.5 eq), and oxalyl chloride (103.26 mg, 813.57 µmol, 71.22 µL, 4 eq) were added to anhydrous dichloromethane (1 mL). The reaction solution was stirred at 20°C for 1 h, and LCMS (quenched with methanol) showed that the reaction was completed. The reaction solution was concentrated under reduced pressure to give the intermediate 5 (100 mg, crude product) which was a white solid. LCMS: rt=0.717 min, 506.3 [M+H]+ (mass of methyl ester), purity: 82.696%.

### Step 4: Synthesis of compound 11

The intermediate 5 (100 mg, 196.04 µmol, 1 eq) was added to pyridine (0.5 mL), followed by adding a mixture of compound 3 (73.30 mg, 294.06 µmol, 1.5 eq) dissolved in pyridine (0.5 mL). The reaction solution was stirred at 0°C for 3 h. LCMS showed that the reaction was completed. The reaction solution was concentrated under reduced pressure and purified by high performance liquid chromatography: column: Phenomenex luna C18 150*25mm*10um, mobile phase: [water (FA)-acetonitrile], gradient: 63%-93% B, 18 min, to obtain compound 11 (9.2 mg, 14.45 µmol, yield 7.37%, purity 97.8%) which was a grayish-white solid. LCMS(39): rt=0.625 min, 623.4 [M+H]⁺, purity: 97.813%. HPLC(39): rt=2.305 min, purity 97.949%. ¹H NMR (400 MHz, DMSO-d6) δ=12.63 (s, 1H), 11.26 (br s, 1H), 8.67 (s, 1H), 6.86 (br d, J=1.6 Hz, 1H), 6.49 (br s, 1H), 6.27 (s, 1H), 6.20 (d, J=2.0 Hz, 1H), 6.10 (br d, J=2.8 Hz, 1H), 3.02-2.90 (m, 2H), 2.09-1.99 (m, 1H), 1.90-1.81 (m, 2H), 1.79-1.61 (m, 5H), 1.44 (s, 5H), 1.35-1.23 (m, 7H), 1.18 (s, 3H), 1.14-1.03 (m, 4H), 1.01-0.89 (m, 8H), 0.89-0.83 (m, 1H).

### Example 12

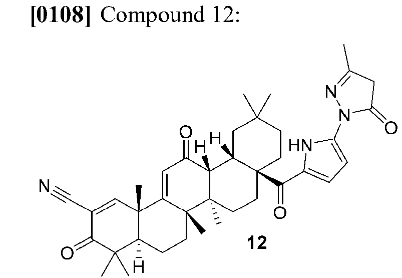

### Synthesis route:

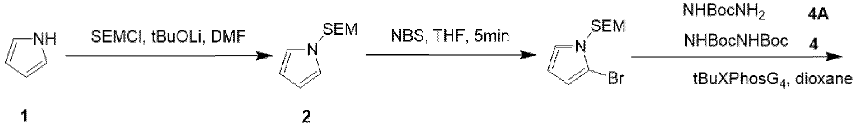

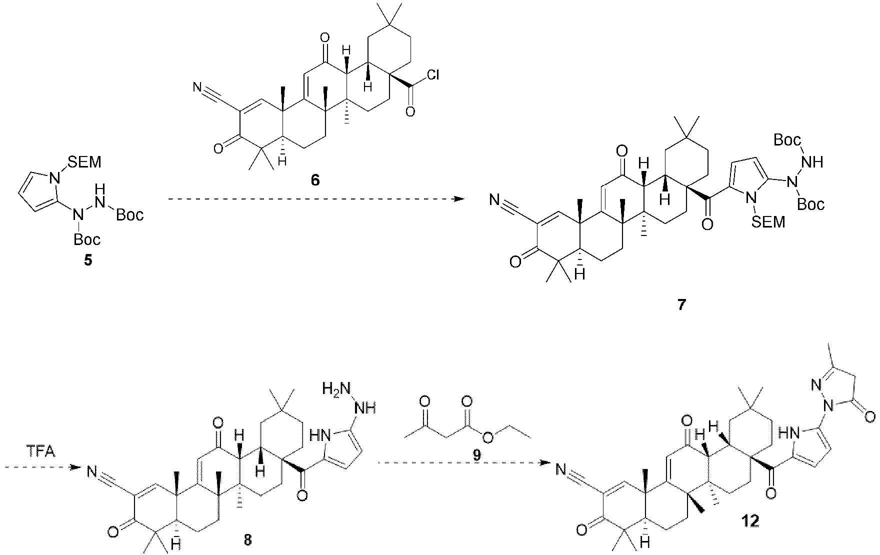

### Example 13

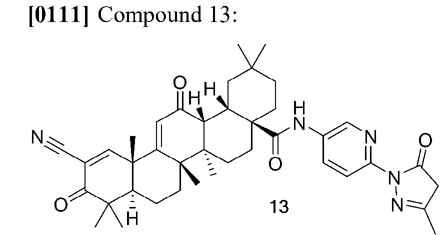

Synthesis route:

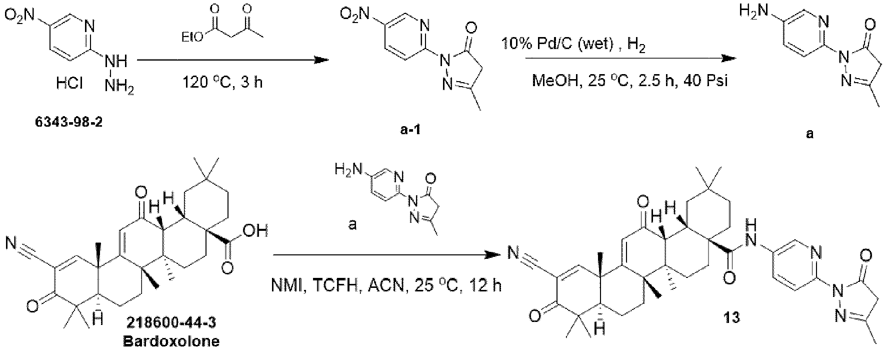

### Step 1: Synthesis of intermediate a-1

Ethyl 3-oxobutyrate (1.37 g, 10.6 mmol, 1.34 mL, 1.00 eq) was added to the solution of material 6343-98-2 (2.00 g, 10.6 mmol, 1.00 eq). The mixture was stirred at 120°C for 2 h. LC-MS (EB11687-1-P1A1) showed a new peak on LC-MS and detected approximately 71.0% of the desired compound. The reaction mixture was concentrated under reduced pressure to obtain a residue. The crude product was ground with ethanol (3 V) at 25°C for 1 h, and filtered, and the solid was collected to give the intermediate a-1 (1.70 g, 7.58 mmol, yield 72.0%, purity 98.2%) which was a white solid. **LCMS:** (EB11687-1-P1A1_LCMS_SH), RT=1.220 min, MS (ESI) m/z=220 [M]⁺. **LCMS:** (EB11687-1-P1C1_LCMS_SH), RT=1.198 min, MS(ESI) m/z=220 [M] ⁺.

### Step 2: Synthesis of intermediate a

Under nitrogen atmosphere, Pd/C (246 mg, 232 µmol, 238 µL, purity 10%, 0.04 eq) was added to a MeOH solution (15.0 mL) of the intermediate a-1 (1.50 g, 6.81 mmol, 1.00 eq). The suspension was degassed and purged with H₂ three times. The mixture was stirred for 2.5 h under conditions of 25°C and H₂ (40.0 psi.). LC-MS (EB11687-2-P1A2) showed a new peak, and detected approximately 85.9% of the expected compound. The reaction mixture was filtered through a diatomaceous earth bed to remove Pd-C, and the diatomaceous earth bed was thoroughly washed with methanol containing 5.00% acetic acid. The filtrates were combined, and evaporated under vacuum to remove solvent. The remaining syrup was suspended in ethyl acetate (10.0 mL) and diluted with hexane (50.0 mL). A yellow crystalline solid suspension was obtained, stirred for 10 min, and filtered to obtain the solid which was washed with hexane, and dried to give yellow intermediate a solid (1.25 g, 6.57 mmol, yield 96.4%). **LCMS:** (EB11687-2-P1A2_LCMS_SH), RT=0.431 min, MS (ESI) m/z=190 [M]⁺.

### Step 3: Synthesis of compound 13

Material 218600-44-3 (500 mg, 1.02 mmol, 1.00 eq), intermediate a (193 mg, 1.02 mmol, 1.00 eq), and NMI (292 mg, 3.56 mmol, 284 µL, 3.50 eq) in MeCN (8.00 mL) was mixed, and then TCFH (N,N,N',N'-Tetramethylchloroformamidinium hexafluorophosphate, 343 mg, 1.22 mmol, 1.20 eq) was added. The mixture was then stirred for 12 h at 25°C under nitrogen atmosphere. LC-MS (EB11687-29-P1A1) showed that approximately 35.6% of expected compound was detected. The residue was concentrated under reduced pressure to obtain the crude product. The crude product was purified by reversed-phase HPLC column: Welch Xtimate C18 40*200 mm 7 µm; mobile phase: [water (NH₄HCO₃)-ACN]; gradient: 56.0%-96.0% B, 25 min to give compound **13,** white solid (273 mg, 262 µmol, yield 39.3%). **LCMS:** (EB11687-29-P1A1_LCMS_SH) RT=1.855 min, MS (ESI) m/z=663.9 [M+1]+. **LCMS:** (EB11687-35-P2C1_LCMS_SH) RT=2.484 min, MS (ESI) m/z=663.3 [M]⁺. **HPLC:** (EB11687-35-P1C4) RT=4.362 min. **1HNMR:** (EB11687-35-P1N2) (CDCl3, 400 MHz) δ ppm 0.92 (s, 3 H) 0.97-1.01 (m, 8 H) 1.08-1.14 (m, 1 H) 1.16 (s, 3 H) 1.17-1.27 (m, 5 H) 1.27-1.45 (m, 4 H) 1.54-1.77 (m, 10 H) 1.90-2.00 (m, 3H) 2.31 (s, 3H) 2.75 (d, *J*=4.80 Hz, 1H) 3.02-3.11 (m, 1H) 5.94 (s, 2H) 6.65 (d, *J*=8.40 Hz, 2H) 7.22 (d, *J*=8.80 Hz, 2H) 8.05 (s, 1H).

### Example 14

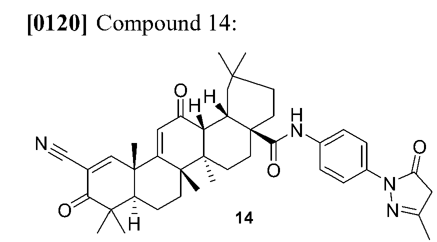

### Synthesis route:

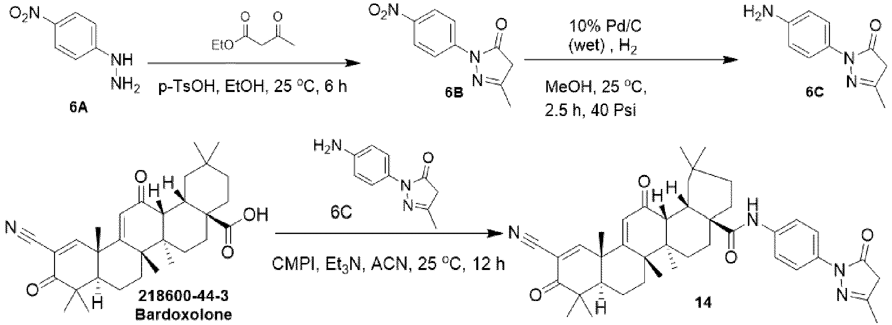

### Synthesis of intermediate 6B

p-TsOH (124 mg, 718 µmol, 0.05 eq) was added to the EtOH (48.0 mL) solution of material 6A (2.0 g, 13.1 mmol, 1.00 eq) and ethyl 3-oxobutyrate (1.95 g, 15.0 mmol, 1.90 mL, 1.15 eq). The mixture was stirred at 80°C for 6 h. LC-MS (EB11687-6-P1 C3) showed a new peak appearing on LC-MS, and detected approximately 96.4% of the expected compound. The mixture was filtered, and the filtrate was concentrated to give a crude product. The crude product was ground in ethanol (4.50 mL) at 25°C to give the intermediate 6B (1.50 g, 6.60 mmol, yield 50.5%, purity 96.4%) which was a red solid. LCMS: (EB11687-6-P1C3_LCMS_SH): RT=0.848 min, MS (ESI) m/z=220 [M+1]+.

### Step 2: Synthesis of intermediate 6C

Under nitrogen atmosphere, Pd/C (124 mg, 116 µmol, purity 10.0%, 0.04 eq) was added to the MeOH (1.00 mL) solution of the intermediate 6B (750 mg, 3.42 mmol, 1.00 eq). The suspension was degassed and purged three times with H₂. The mixture was stirred for 2.5 h under conditions of 25°C and H₂ (40.0 psi). LC-MS (EB11687-14-P1A1) showed that approximately 96.0% of desired compound was detected. The reaction mixture was filtered through a diatomaceous earth bed to remove Pd-C, and the diatomaceous earth bed was thoroughly washed with methanol containing 5% acetic acid. The filtrates were combined, and evaporated under vacuum to remove solvent. The residual syrup was suspended in ethyl acetate (10.0 mL) and diluted with hexane (50.0 mL) to obtain a yellow crystalline solid suspension, stirred for 10 min, and filtered to obtain the solid which was washed with hexane and dried to give a red intermediate 6C solid (500 mg, 2.64 mmol, yield 77.2%). **LCMS:** (EB11687-14-P1A1_LCMS_SH): RT=0.154 min, MS (ESI) *m*/*z*=189.9 [M+1]+.

Step 3: Synthesis of compound 14

CMPI (2-chloro-1-methylpyridinium iodide, 338 mg, 1.32 mmol, 1.30 eq) and Et₃N (309 mg, 3.05 mmol, 425 µL, 3.00 eq) were added to the acetonitrile (5.00 mL) solution of material 218600-44-3 (500 mg, 1.02 mmol, 1.00 eq) and intermediate 6C (192 mg, 1.02 mmol, 1.00 eq). The mixture was stirred at 25°C for 3 h. LC-MS (EB11687-40-P1A3) showed appearance of a new peak and detected approximately 20% of the desired compound. The mixture was concentrated to obtain a crude product. The crude product was purified by reversed-phase HPLC (chromatography column: Welch Xtimate C18 40*200 mm 7 µm; mobile phase: [water (NH₃H₂O + NH₄HCO₃)-ACN]; gradient: 54.0%-94.0% B in 25 min) to give the compound 14 (74.0 mg, 112 µmol, 11.0% yield) which was a brown solid. **LCMS:** (EB11687-40-P1A3_LCMS_SH): RT=1.834 min, MS (ESI) m/z=663 [M+1]⁺; **LCMS:** (EB11687-41-P1C1_LCMS_SH): RT=2.576 min, MS (ESI) m/z=663 [M+1]⁺; **HPLC:** (EB11687-41-P1C3): RT=4.377 min. **¹H NMR:** (EB11687-41-P1N1) (CDCl₃, 400 MHz) δ ppm 0.95 (s, 3H) 1.00-1.04 (m, 9H) 1.18 (s, 3H) 1.27 (s, 3H) 1.33 (br d, *J*=2.38 Hz, 2H) 1.45 (d, *J*=13.51 Hz, 2H) 1.50 (s, 3H) 1.72-1.82 (m, 10H) 1.92-2.02 (m, 3H) 2.34 (s, 3H) 2.77 (d, *J* =4.63 Hz, 1H) 3.03-3.17 (m, 1 H) 5.97 (s, 2H) 6.71 (d, *J* =8.25 Hz, 2H) 7.27 (s, 2H) 8.06-8.10 (m, 1H) .

### Example 15

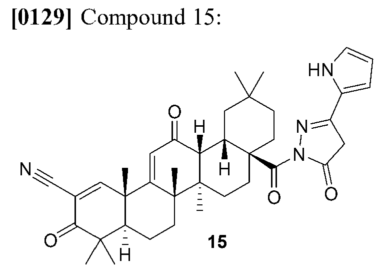

### Synthesis route:

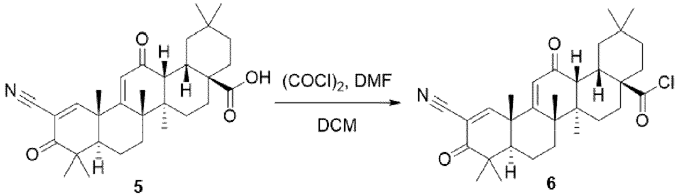

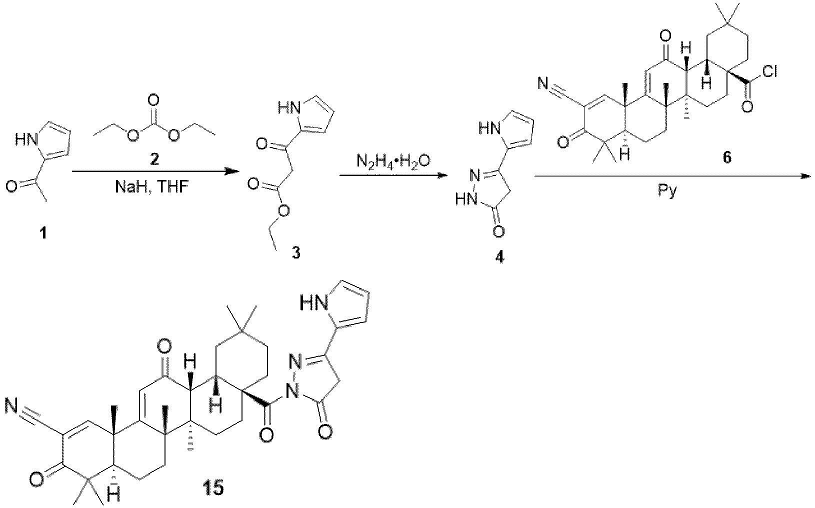

### Synthesis of intermediate 3

The compound 1 (5 g, 40.60 mmol, 4.81 mL, 1 eq) was dissolved in anhydrous tetrahydrofuran (50 mL), followed by portionwise adding sodium hydride (1.95 g, 48.72 mmol, purity 60%, 1.2 eq) under nitrogen condition at 20°C, and reacting at 0°C for 0.5 h. Then, the compound 2 (5.28 g, 44.66 mmol, 5.41 mL, 1.1 eq) was added dropwise to the reaction system under nitrogen protection, for reaction at 20°C for 2 h. TLC showed that most of the raw material reacted and a new spot was formed. The reaction solution was slowly poured into a saturated ammonium chloride aqueous solution (200 mL), and then extracted three times with ethyl acetate (100 mL*3). The combined organic phase was washed once with saturated brine (150 mL), dried over anhydrous sodium sulfate, filtered, and concentrated to obtain the crude product. The crude product was purified by normal-phase column chromatography (petroleum ether/ethyl acetate=1/0~10/1) to give the intermediate 3 (4 g, 20.49 mmol, yield 50.47%) which was yellow oily. ¹H NMR (400 MHz, CHLOROFORM-d) δ=6.97 (dd, J=1.6, 4.2 Hz, 1H), 6.85 (s, 1H), 6.15 (dd, J=2.4, 4.2 Hz, 1H), 4.21 (q, J=7.2 Hz, 2H), 3.95 (s, 3H), 3.81 (s, 2H), 1.28 (t, J=7.2 Hz, 3H).

### Step 2: Synthesis of intermediate 4

The intermediate 3 (2 g, 10.25 mmol, 1 eq) was dissolved in anhydrous ethanol (20 mL), followed by adding hydrazine hydrate (5.23 g, 102.45 mmol, 5.07 mL, purity 98%, 10 eq) at 20°C, and then reacting at 80°C for 2 h. After LCMS detected that the raw material reacted completely, the reaction solution was concentrated and then isolated and purified by reverse-phase column [220 g, water (formic acid)-acetonitrile system], and then lyophilized to obtain the intermediate 4 (900 mg, 5.52 mmol, yield 53.84%, purity 100%), which was yellow oily. LCMS: rt=0.418 min, 164.1 [M+H]⁺. ¹H NMR (400 MHz, DMSO-d6) δ=8.45 (s, 1H), 6.77 (s, 1H), 6.27 (dd, J=1.8, 3.6 Hz, 1H), 6.00 (dd, J=2.8, 3.6 Hz, 1H), 5.56 (s, 1H), 3.68 (s, 3H).

### Step 3: Synthesis of intermediate 6

The compound 5 (300 mg, 610.18 µmol, 1 eq) was dissolved in anhydrous dichloromethane (5 mL), followed by adding N,N-dimethylformamide (4.46 mg, 61.02 µmol, 4.69 µL, 0.1 eq) and oxaloyl chloride (232.34 mg, 1.83 mmol, 160.24 µL, 3 eq) at 20°C, purging with nitrogen three times, and then reacting at 20°C for 1 h. After LCMS detected that the raw material reacted completely (small sample dissolved in methanol for detection), the reaction solution was concentrated to give the intermediate 6 (300 mg, 588.11 µmol, yield 96.38%) which was yellow oily. LCMS: rt=0.608 min, 506.4 [M+H]+ (Ms of methyl ester).

### Step 4: Synthesis of compound 15

The intermediate 4 (95.97 mg, 588.11 µmol, 1 eq) and intermediate 6 (300 mg, 588.11 µmol, 1 eq) were dissolved in dichloromethane (5 mL), followed by adding pyridine (69.78 mg, 882.17 µmol, 71.20 µL, 1.5 eq), reacting at 20°C for 16 h. LCMS monitoring showed that the raw material was consumed completely and a product was formed. The reaction solution was rotary evaporated to dryness, and then isolated and purified by reverse-phase preparation [water (formic acid)-acetonitrile system] and then lyophilized to obtain compound **15** (22.51 mg, 35.590 µmol, yield 60.70%, purity 99.78%) which was a white solid. Prep -HPLC: column: Phenomenex luna C18 150*40mm*15um; mobile phase: [water(FA)-ACN]; gradient: 62%-92% B over 15 min. LCMS: rt=1.741 min, 637.4 [M+H]⁺. HPLC: rt=2.144 min. ¹H NMR (400 MHz, CHLOROFORM-d) δ=8.05 (s, 1H), 7.27 (s, 1H), 6.78-6.73 (m, 1H), 6.38 (dd, *J*=1.4, 3.4 Hz, 1H), 6.22-6.15 (m, 2H), 5.98 (s, 1H), 3.72 (s, 3H), 3.20-3.13 (m, 2H), 2.13-2.02 (m, 2H), 1.98-1.86 (m, 2H), 1.85-1.75 (m, 5H), 1.67 (br d, *J*=13.2 Hz, 1H), 1.60-1.54 (m, 1H), 1.51-1.47 (m, 3H), 1.43-1.37 (m, 4H), 1.35-1.24 (m, 6H), 1.18 (s, 3H), 1.06 (d, *J*=1.6 Hz, 6H), 0.97-0.92 (m, 3H).

### Example 16

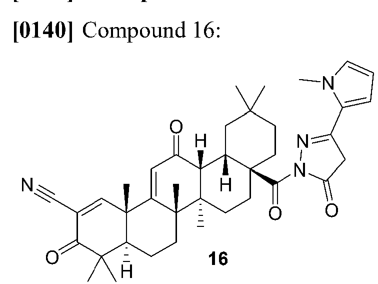

### Synthesis route:

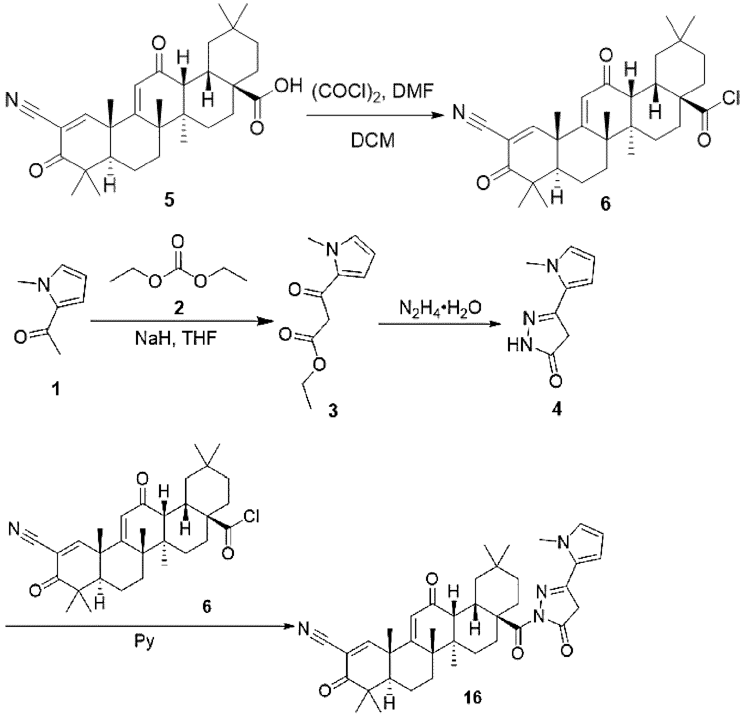

### Synthesis of intermediate 3

The compound 1 (5 g, 40.60 mmol, 4.81 mL, 1 eq) was dissolved in anhydrous tetrahydrofuran (50 mL), followed by portionwise adding sodium hydride (1.95 g, 48.72 mmol, purity 60%, 1.2 eq) under nitrogen condition at 20°C, reacting at 0°C for 0.5 h, then adding dropwise the compound 2 (5.28 g, 44.66 mmol, 5.41 mL, 1.1 eq) to the reaction system under nitrogen protection, and reacting at 20°C for 2 h. TLC showed that most of the raw material reacted and a new spot was formed. The reaction solution was slowly poured into a saturated ammonium chloride aqueous solution (200 mL), and then extracted three times with ethyl acetate (100 mL*3). The combined organic phase was washed once with saturated brine (150 mL), dried over anhydrous sodium sulfate, filtered, and concentrated to obtain the crude product. The crude product was purified by normal-phase column chromatography (petroleum ether/ethyl acetate=1/0~10/1) to give the intermediate 3 (4 g, 20.49 mmol, yield 50.47%) which was yellow oily. ¹H NMR (400 MHz, CHLOROFORM-d) δ=6.97 (dd, J=1.6, 4.2 Hz, 1H), 6.85 (s, 1H), 6.15 (dd, J=2.4, 4.2 Hz, 1H), 4.21 (q, J=7.2 Hz, 2H), 3.95 (s, 3H), 3.81 (s, 2H), 1.28 (t, J=7.2 Hz, 3H).

### Step 2: Synthesis of intermediate 4

The intermediate 3 (2 g, 10.25 mmol, 1 eq) was dissolved in anhydrous ethanol (20 mL), followed by adding hydrazine hydrate (5.23 g, 102.45 mmol, 5.07 mL, purity 98%, 10 eq) at 20°C, then reacting at 80°C for 2 h. After LCMS detected that the raw material reacted completely, the reaction solution was concentrated and then isolated and purified by reverse-phase column [220 g, water (formic acid)-acetonitrile system] and then lyophilized to obtain the intermediate 4 (900 mg, 5.52 mmol, yield 53.84%, purity 100%) which was yellow oily. LCMS: rt=0.418 min, 164.1 [M+H]⁺. ¹H NMR (400 MHz, DMSO-d6) δ=8.45 (s, 1H), 6.77 (s, 1H), 6.27 (dd, J=1.8, 3.6 Hz, 1H), 6.00 (dd, J=2.8, 3.6 Hz, 1H), 5.56 (s, 1H), 3.68 (s, 3H).

### Step 3: Synthesis of intermediate 6

The compound 5 (300 mg, 610.18 µmol, 1 eq) was dissolved in anhydrous dichloromethane (5 mL), followed by adding N,N-dimethylformamide (4.46 mg, 61.02 µmol, 4.69 µL, 0.1 eq) and oxaloyl chloride (232.34 mg, 1.83 mmol, 160.24 µL, 3 eq) at 20°C, purging with nitrogen three times, and then reacting at 20°C for 1 h. After LCMS detected that the raw material reacted completely (small sample dissolved in methanol for detection), the reaction solution was concentrated to give the intermediate 6 (300 mg, 588.11 µmol, yield 96.38%) which was yellow oily. LCMS: rt=0.608 min, 506.4 [M+H]+ (Ms of methyl ester).

### Step 4: Synthesis of compound 16

The intermediate 4 (95.97 mg, 588.11 µmol, 1 eq) and intermediate 6 (300 mg, 588.11 µmol, 1 eq) were dissolved in dichloromethane (5 mL), followed by adding pyridine (69.78 mg, 882.17 µmol, 71.20 µL, 1.5 eq), and reacting at 20°C for 16 h. LCMS monitoring showed that the raw material was consumed completely and a product was formed. The reaction solution was rotary evaporated to dryness, and then isolated and purified by reverse-phase preparation [water (formic acid)-acetonitrile system] and then lyophilized to obtain the compound 16 (227.1 mg, 355.83 µmol, yield 60.50%, purity 99.78%) which was a white solid. Prep-HPLC: column: Phenomenex luna C18 150*40mm*15um; mobile phase: [water(FA)-ACN]; gradient:62%-92% B over 15 min. LCMS: rt=1.741 min, 637.4 [M+H]⁺. HPLC: rt=2.144 min. ¹H NMR (400 MHz, CHLOROFORM-d) δ=8.05 (s, 1H), 7.27 (s, 1H), 6.78-6.73 (m, 1H), 6.38 (dd, J=1.4, 3.4 Hz, 1H), 6.22-6.15 (m, 2H), 5.98 (s, 1H), 3.72 (s, 3H), 3.20-3.13 (m, 2H), 2.13-2.02 (m, 2H), 1.98-1.86 (m, 2H), 1.85-1.75 (m, 5H), 1.67 (br d, J=13.2 Hz, 1H), 1.60-1.54 (m, 1H), 1.51-1.47 (m, 3H), 1.43-1.37 (m, 4H), 1.35-1.24 (m, 6H), 1.18 (s, 3H), 1.06 (d, J=1.6 Hz, 6H), 0.97-0.92 (m, 3H).

### Example 17

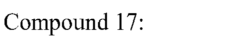

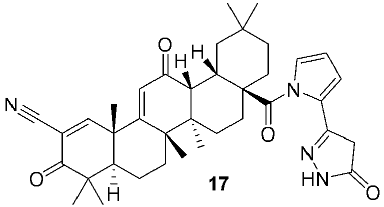

### Synthesis route:

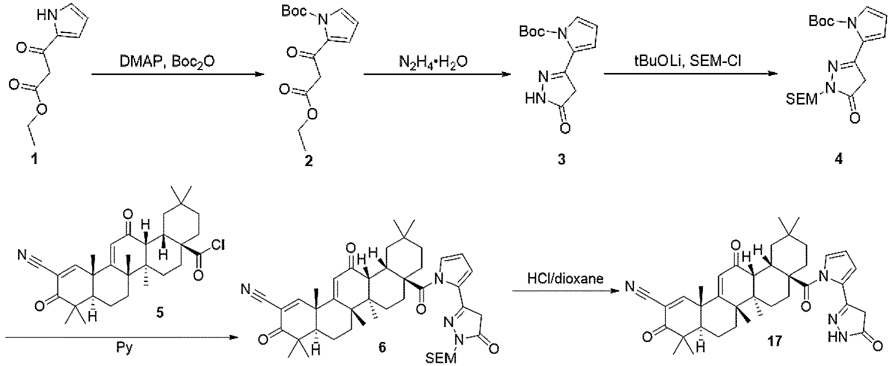

### Step 1: Synthesis of intermediate 2

The compound 1 (1 g, 5.52 mmol, 1 eq), triethylamine (614.32 mg, 6.07 mmol, 845.01 µL, 1.1 eq), Boc2O (1.32 g, 6.07 mmol, 1.39 mL, 1.1 eq), and DMAP (67.43 mg, 551.91 µmol, 0.1 eq) were added to anhydrous tetrahydrofuran (20 mL). The reaction solution was stirred at 20°C for 14 h. LCMS showed that the reaction was completed. The reaction solution was concentrated under reduced pressure to obtain a crude product. The crude product was purified by column chromatography (silica, petroleum ether:ethyl acetate=1:0~4:1) to give the intermediate 2 (1.1 g, 3.44 mmol, yield 62.35%, purity 88%) which was yellow oily. LCMS: rt=0.524 min, 304.1 [M+Na]+, purity 87.557%.

### Step 2: Synthesis of intermediate 3

The compound 2 (1.1 g, 3.91 mmol, 1 eq) and hydrazine hydrate (4.27 g, 83.59 mmol, 4.14 mL, purity 98%, 21.38 eq) were added to anhydrous ethanol (15 mL). The reaction solution was heated heated at 80°C for 16 h under a nitrogen atmosphere, and LCMS showed that the reaction was completed. After cooling to room temperature, the reaction solution was concentrated under reduced pressure to give the intermediate 3 (900 mg, 3.61 mmol, 92.33% yield, purity 95%) which was yellow oily. LCMS: rt=0.303 min, 150.0 [M-C5H9O2+H]⁻, purity 94.705%. ¹H NMR (400 MHz, DMSO-d6) δ=11.21 (s, 1H), 6.70 (s, 1H), 6.23 (br d, J=2.0 Hz, 1H), 6.00 (t, J=2.8 Hz, 1H), 3.41 (br s, 2H), 1.36 (s, 9H).

### Step 3: Synthesis of intermediate 4

The intermediate 3 (1.7 g, 6.82 mmol, 1 eq) was dissolved in anhydrous N,N-dimethylformamide (25 mL), followed by adding lithium tert-butoxide (818.92 mg, 10.23 mmol, 922.20 µL, 1.5 eq) at 0°C, reacting at 0°C for 0.5 h, then adding 2-(trimethylsilyl)ethoxymethyl chloride (1.36 g, 8.18 mmol, 1.45 mL, 1.2 eq) at 0°C, and then reacting at 20°C for 1.5 h. After LCMS detected that the raw material reacted completely, the reaction solution was poured into a saturated ammonium chloride aqueous solution (100 mL), and then extracted three times with ethyl acetate (70 mL*3). The combined organic phase was washed three times with saturated brine (70 mL), dried over anhydrous sodium sulfate, filtered, and concentrated to obtain the crude product. The crude product was purified by normal-phase column chromatography (petroleum ether/ethyl acetate=0-50%) to give the intermediate 4 (610 mg, 1.45 mmol, yield 21.21%, purity 90%) which was a yellow solid. LCMS: rt=0.557 min, 280.3 [M-Boc+H]⁺.

### Step 4: Synthesis of intermediate 6

The intermediate 4 (368.29 mg, 970.39 µmol, 1 eq) and intermediate 5 (495 mg, 970.39 µmol, 1 eq) were dissolved in dichloromethane (10 mL), followed by adding pyridine (230.27 mg, 2.91 mmol, 234.97 µL, 3 eq), reacting at 20°C for 16 h. LCMS monitoring showed that the raw material was consumed completely and a product was formed. The reaction solution was rotary evaporated to dryness and purified by normal-phase column chromatography (petroleum ether/ethyl acetate=0-50%) to give the compound 6 (437 mg, 580.30 µmol, yield 59.80%) which was a yellow solid. LCMS: rt=0.734 min, 753.6 [M+H]⁺.

### Step 5: Synthesis of compound 17

The intermediate 6 (100 mg, 132.79 µmol, 1 eq) was dissolved in hydrochloric acid/1,4-dioxane (5 mL, 2 M) and heated to 50°C for reaction for 2 h. LCMS monitoring showed that the raw material was consumed completely and a product was formed. The reaction solution was rotary evaporated to dryness, adjusted to be alkaline with sodium bicarbonate aqueous solution and then extracted three times with ethyl acetate (10 mL*3). The combined organic phase was washed once with saturated brine (15 mL), dried over anhydrous sodium sulfate, and rotary evaporated to dryness to obtain the crude product. The crude product was isolated and purified by reverse-phase preparation [water (formic acid)-acetonitrile system] and then lyophilized to obtain the compound 17 (6.2 mg, 9.75 µmol, yield 7.34%, purity 97.96%) which was a white solid. Prep-HPLC: column: Phenomenex luna C18 150*25mm*10um; mobile phase: [water (FA)-ACN]; gradient: 55%-85% B over 10 min. LCMS: rt=1.664 min, 623.4 [M+H]⁺. HPLC: rt=2.043 min. ¹H NMR (400 MHz, CHLOROFORM-d) δ=8.78-8.63 (m, 1H), 8.04 (s, 1H), 6.89 (br s, 1H), 6.46 (br s, 1H), 6.29 (br d, J=2.8 Hz, 1H), 6.17 (s, 1H), 5.98 (s, 1H), 3.19-3.10 (m, 2H), 2.64 (s, 1H), 2.06-2.01 (m, 2H), 1.95-1.88 (m, 2H), 1.78 (br s, 5H), 1.67 (br d, J=13.2 Hz, 1H), 1.55 (br d, J=12.8 Hz, 1H), 1.48 (s, 3H), 1.36 (s, 4H), 1.32-1.26 (m, 6H), 1.18 (s, 3H), 1.05 (d, J=4.2 Hz, 6H), 0.95 (s, 3H) .

### Example 18

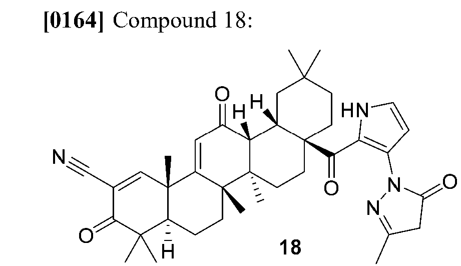

### Synthesis route:

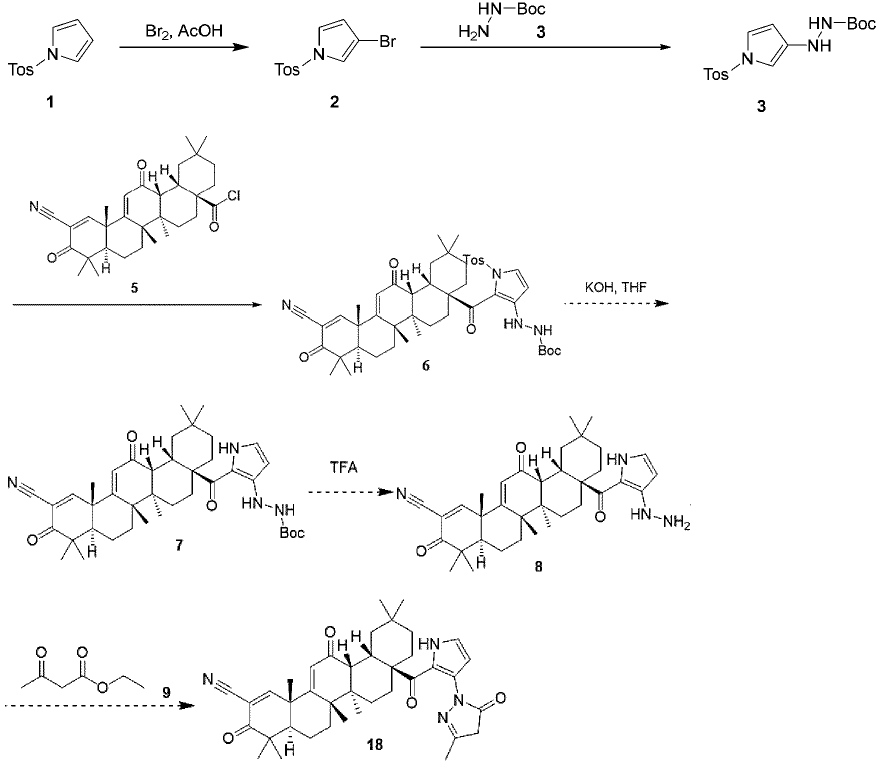

### Example 19

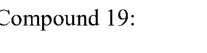

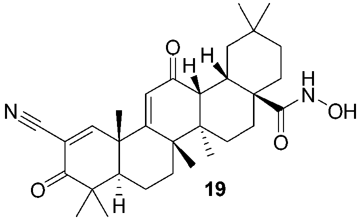

### Synthesis route:

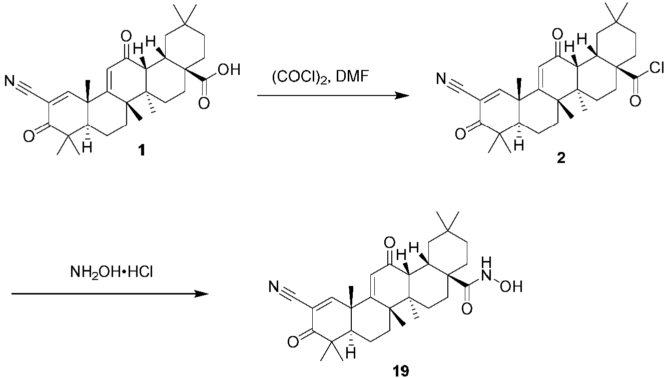

### Step 1: Synthesis of intermediate 2

N,N-dimethylformamide (7.43 mg, 101.70 µmol, 7.82 µL, 0.1 eq) and oxaloyl chloride (387.25 mg, 3.05 mmol, 267.07 µL, 3 eq) were added to the dichloromethane (8 mL) solution of material 1 (500 mg, 1.02 mmol, 1 eq). The reaction solution reacted at 25°C for 1 h. LCMS monitoring showed that the reaction raw material disappeared completely and the main peak was the target product. The reaction solution was concentrated under reduced pressure to obtain the intermediate 2 (500 mg, 980.19 µmol, 96.38% yield) which was a faint yellow solid. LCMS: Rt=0.917 min, 510.4 [M+H]+ ESI pos.

### Step 2: Synthesis of compound 19

At 0°C, the dichloromethane (5 mL) solution of intermediate 2 (500 mg, 980.19 µmol, 1 eq) was added to the dichloromethane (5 mL) solution of the compound hydroxylamine hydrochloride (81.74 mg, 1.18 mmol, 1.2 eq) and N,N-diisopropylethylamine (380.04 mg, 2.94 mmol, 512.18 µL, 3 eq). The reaction solution was heated to 20°C for reaction for 2 h. LCMS monitoring showed that the raw material was completely consumed and a product was formed. The reaction solution was filtered and concentrated. The crude product was isolated and purified by reverse-phase preparation [water (formic acid)-acetonitrile system] and then lyophilized to obtain the compound **19** (136.20 mg, 266.39 µmol, yield 27.18%, purity 99.1%). Prep-HPLC: column: Phenomenex luna C18 150*40mm*15um; mobile phase: [water(FA)-ACN]; gradient:45%-75% B over 15 min. LCMS: Rt=1.339 min, 507.3 [M+H]+ ESI pos. HPLC: Rt=1.816 min. ¹H NMR (400 MHz, CHLOROFORM-d) δ=9.39-9.07 (m, 1H), 8.03 (s, 1H), 6.04 (s, 1H), 2.97 (d, J=4.6 Hz, 1H), 2.92-2.84 (m, 1H), 2.01-1.92 (m, 1H), 1.85-1.79 (m, 1H), 1.78-1.71 (m, 4H), 1.70-1.62 (m, 3H), 1.57-1.45 (m, 4H), 1.42 (s, 3H), 1.31 (br d, J=4.0 Hz, 1H), 1.28 (s, 3H), 1.26-1.23 (m, 1H), 1.21 (s, 3H), 1.12 (s, 3H), 0.97 (d, J=5.8 Hz, 6H), 0.88 (s, 3H).

### Example 20

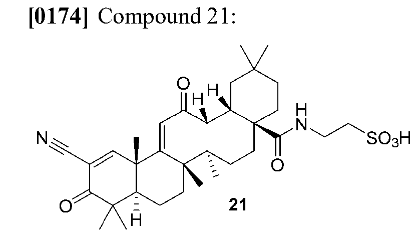

### Synthesis route:

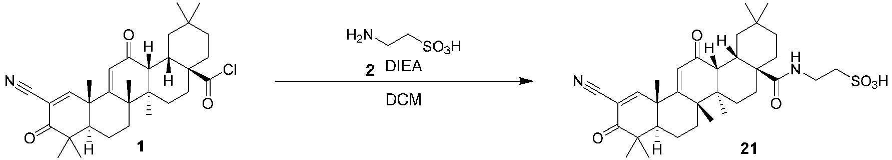

### Step 1: Synthesis of compound 21

The material 2 (24.53 mg, 196.04 µmol, 24.44 µL, 1 eq) was added to anhydrous dichloromethane (1 mL), followed by adding N,N-diisopropylethylamine (126.68 mg, 980.19 µmol, 170.73 µL, 5 eq). The reaction mixture was cooled to 0°C, and slowly added with material 1 (100.00 mg, 196.04 µmol, 1 eq) dissolved in anhydrous dichloromethane (1 mL). The reaction solution was stirred at 25°C for 12 h. After LCMS detected that the reaction was completed, the reaction solution was concentrated to obtain a crude product. The crude product was purified by reverse-phase preparation (C18 column, 0.1% trifluoroacetic acid solution), and lyophilized to obtain the crude product. The crude product was purified by reverse-phase preparation (C18 column, 0.1% hydrogen chloride solution) and lyophilized to obtain the compound **21** (5.0 mg, off-white solid, yield 4.26%). Prep-HPLC (column: Phenomenex luna C18 150*25mm*10um; mobile phase: [water(TFA)-ACN]; gradient: 40%-70% B over 10 min). LCMS: rt=1.225 min, 599.3 [M+H]⁺, purity 100%. HPLC: retention time=1.724 min, purity 97.989%. ¹H NMR: 1H NMR (400 MHz, DMSO-d6) δ=8.65 (s, 1H), 7.88-7.76 (m, 1H), 6.19 (s, 1H), 3.47-3.36 (m, 1H), 3.35-3.24 (m, 1H), 3.03-2.97 (m, 1H), 2.90-2.80 (m, 1H), 2.57-2.53 (m, 2H), 1.98-1.79 (m, 3H), 1.64 (br s, 3H), 1.63-1.47 (m, 3H), 1.44-1.43 (m, 3H), 1.42-1.36 (m, 2H), 1.36-1.27 (m, 2H), 1.27-1.24 (m, 3H), 1.19-1.16 (m, 3H), 1.16-1.08 (m, 2H), 1.06 (s, 3H), 0.93 (s, 3H), 0.89 (s, 3H), 0.86 (s, 3H) .

### Example 21

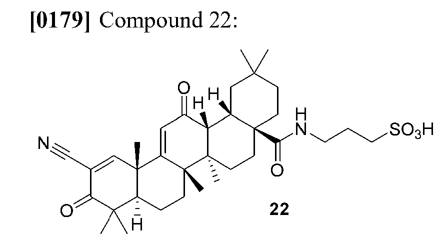

### Synthesis route:

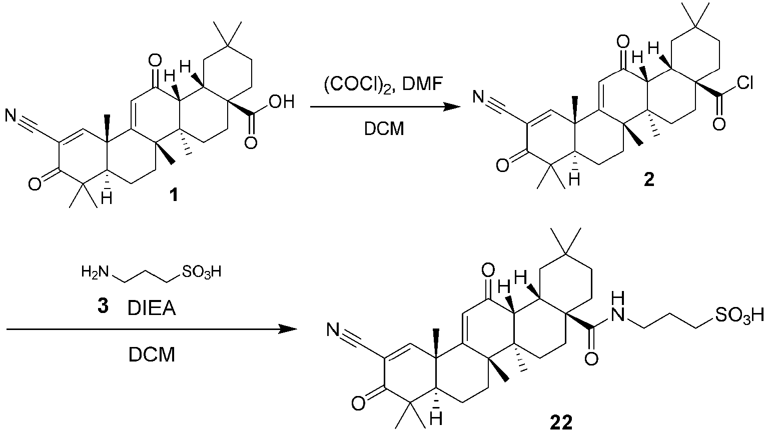

### Step 1: Synthesis of intermediate 2

The material 1 (200 mg, 406.79 µmol, 1 eq) and N,N-dimethylformamide (2.97 mg, 40.68 µmol, 3.13 µL, 0.1 eq) were added and dissolved in anhydrous dichloromethane (1.2 mL), followed by purging with nitrogen three times, then cooling to 0°C, adding oxalyl chloride (154.89 mg, 1.22 mmol, 106.82 µL, 3 eq) dissolved in anhydrous dichloromethane (0.8 mL), and stirring at 25°C for 1 h. The reaction solution was concentrated under reduced pressure to obtain a crude product. The crude product of the intermediate 2 (200 mg, crude) was used directly in the next step reaction.

### Step 2: Synthesis of compound 22

3-Amino-1-propanesulfonic acid (27.28 mg, 196.04 µmol, 1 eq) was added to anhydrous dichloromethane (1 mL), followed by adding N,N-diisopropylethylamine (126.68 mg, 980.19 µmol, 170.73 µL, 5 eq). The reaction solution was cooled to 0°C, and the intermediate 2 (100 mg, 196.04 µmol, 1 eq) dissolved in anhydrous dichloromethane (1 mL) was slowly added. The reaction solution was stirred at 25°C for 12 h. After LCMS detected that the reaction was completed, the reaction solution was concentrated to obtain the crude product. The crude product was purified by reverse-phase preparation (C18 column, 0.1% sodium bicarbonate solution), and lyophilized to obtain the crude product. The crude product was purified by reverse-phase preparation (C18 column, 0.1% hydrogen chloride solution), and lyophilized to obtain the compound **22** (9.5 mg, off-white solid, yield 7.91%) was obtained. Prep-HPLC (column: Waters Xbridge 150*25mm*5um; mobile phase: [water(NH₄HCO₃)-ACN]; gradient: 22%-52% B over 9 min). Prep-HPLC (column: Phenomenex luna C18 150*25mm* 10um; mobile phase: [water(HCl)-ACN]; gradient: 35%-65% B over 18 min). LCMS: rt=1.219 min, 613.4 [M+H]⁺, 100% purity. HPLC: retention time=1.708 min, purity 99.318%. ¹H NMR: 1H NMR (400 MHz, DMSO-d6) δ=8.64 (s, 1H), 7.89-7.73 (m, 1H), 6.18 (s, 1H), 3.22-3.12 (m, 1H), 3.12-3.01 (m, 2H), 2.90-2.80 (m, 1H), 2.44-2.39 (m, 2H), 1.92-1.77 (m, 3H), 1.76-1.68 (m, 3H), 1.67-1.48 (m, 5H), 1.47-1.39 (m, 6H), 1.36-1.30 (m, 1H), 1.29-1.25 (m, 1H), 1.24-1.22 (m, 3H), 1.18-1.15 (m, 3H), 1.15-1.08 (m, 2H), 1.05 (s, 3H), 0.92 (s, 3H), 0.88 (s, 3H), 0.85 (s, 3H).

### Example 22

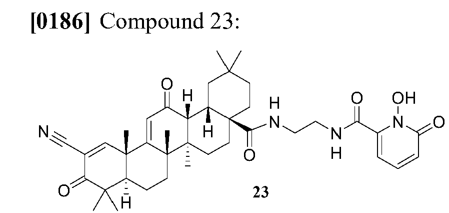

### Synthesis route:

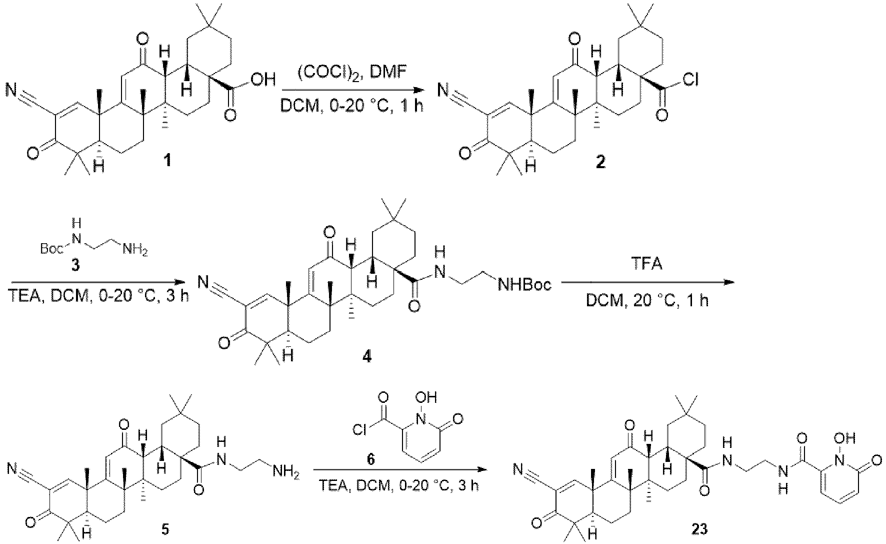

### Step 1: Synthesis of intermediate 2

The material 1 (300 mg, 610.18 µmol, 1 eq) was dissolved in anhydrous dichloromethane (2 mL). One drop of N,N-dimethylformamide was added, and then the reaction solution was cooled in an ice-water bath. Oxaloyl chloride (309.79 mg, 2.44 mmol, 213.65 µL, 4 eq) was dissolved in anhydrous dichloromethane (2 mL) and then slowly added dropwise to the stirred reaction solution. The reaction solution was slowly heated to 20°C and stirred for 1 h. LCMS (quenched with methanol) monitoring showed that the raw material reacted completely. The reaction solution was rotary evaporated and concentrated to give the intermediate 2 (311 mg, crude) which was a white solid. LCMS (quenched with methanol): rt=0.648 min, 506.3 [M+H]⁺.

### Step 2: Synthesis of intermediate 4

The compound **3** (179.86 mg, 914.52 µmol, 177.03 µL, 1.5 eq, HCl) was dissolved in anhydrous dichloromethane (3 mL), followed by adding triethylamine (308.46 mg, 3.05 mmol, 424.30 µL, 5 eq). The reaction solution was cooled in an ice-water bath, and then the solution of the compound **2** (311 mg, 609.68 µmol, 1 eq) dissolved in anhydrous dichloromethane (2 mL) was slowly added dropwise. The reaction solution was slowly heated to 20°C and stirred for 3 h. LCMS monitoring showed that the raw material reacted completely. The reaction mixture was concentrated and then purified by column chromatography (ethyl acetate/petroleum ether=0-51%) to give the intermediate **4** (370 mg, 583.73 µmol, yield 95.74%) which was a colorless gel. **LCMS:** rt=0.619 min, 634.5 [M+H]⁺.

### Step 3: Synthesis of intermediate 5

The intermediate 4 (370 mg, 583.73 µmol, 1 eq) was dissolved in a prepared solution of dichloromethane/trifluoroacetic acid = 5/1 (6 mL) and stirred at 20°C for 1 h. LCMS monitoring showed that the raw material reacted completely. The reaction solution was concentrated to obtain the intermediate 5 (378 mg, crude, TFA) which was a faint yellow gel. LCMS: rt=0.494 min, 534.3 [M+H]⁺.

### Step 4: Synthesis of compound 23

The intermediate 5 (378 mg, 583.54 µmol, 1 eq, TFA) was added sequentially with anhydrous dichloromethane (3 mL) and triethylamine (472.39 mg, 4.67 mmol, 649.77 µL, 8 eq). The reaction solution was cooled in an ice-water bath. The material 6 (202.55 mg, 1.17 mmol, 2 eq) was dissolved in anhydrous dichloromethane (2 mL) and then slowly added dropwise to the stirred reaction solution. The reaction solution was slowly heated to 20°C and stirred for 3 h. LCMS monitoring showed that the raw material reacted completely. The reaction solution was concentrated and then subjected to reverse-phase preparation and lyophilized to obtain a crude product. The crude product was purified by prep-TLC (dichloromethane/methanol=15:1) and lyophilized to obtain the compound **23** (62.8 mg, 89.28 µmol, yield 15.30%, purity 93.92%) which was a yellow solid. HPLC: (1) column: Phenomenex luna C18 150*40mm* 15um; mobile phase: [water(FA)-ACN]; gradient:45%-75% B over 15 min. LCMS: rt=0.630 min, 671.4 [M+H]⁺. HPLC: rt=1.761 min. 1H NMR (400 MHz, METHANOL-d4) δ=8.37 (s, 1H), 7.92-7.75 (m, 1H), 7.53-7.33 (m, 2H), 6.97-6.79 (m, 1H), 6.07 (s, 1H), 3.64 (br d, J=1.0 Hz, 3H), 3.10 (br s, 1H), 3.06-2.97 (m, 1H), 1.90-1.78 (m, 2H), 1.70 (br d, J=1.2 Hz, 4H), 1.62-1.51 (m, 4H), 1.46-1.37 (m, 4H), 1.31 (br s, 2H), 1.24 (br s, 4H), 1.14 (br s, 6H), 1.09 (br s, 2H), 0.94 (br s, 6H), 0.88 (br s, 3H).

### Example 23

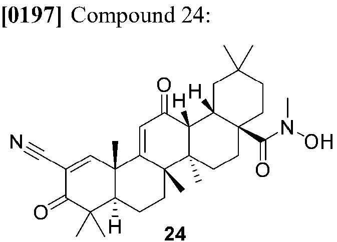

### Synthesis route:

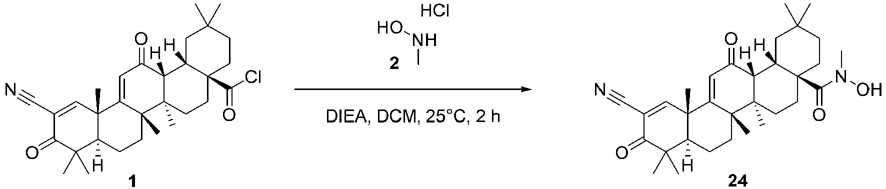

### Step 1: Synthesis of compound 24

N,N-diisopropylethylamine (633.41 mg, 4.90 mmol, 853.65 µL, 5 eq) was added to a dichloromethane (7.5 mL) solution of N-methylhydroxylamine hydrochloride (98.24 mg, 1.18 mmol, 1.2 eq), followed by purging with nitrogen three times, then cooling to 0°C, slowly adding the dichloromethane (7.5 mL) solution of material 1 (500 mg, 980.19 µmol, 1 eq), and stirring at 25°C for 2 h. LCMS monitoring showed that the reaction raw material disappeared completely and the main peak was the target product. The reaction solution was concentrated under reduced pressure to obtain the crude product. The crude product was isolated and purified by reverse-phase preparation [water (formic acid)-acetonitrile system] and lyophilized to obtain the compound 24 (267.5 mg, 500.48 µmol, 51.06% yield, purity 97.42%) which was a white solid. Prep-HPLC (column: Phenomenex luna C18 150*40mm* 15um; mobile phase: [water(FA)-ACN]; gradient:55%-85% B over 15 min). LCMS: Retention time=1.461 min, 521.3 [M+H]⁺. HPLC: retention time=1.831 min, purity 97.42%. 1H NMR (400 MHz, CHLOROFORM-d) δ=8.31 (s, 1H), 8.08 (s, 1H), 5.99 (s, 1H), 3.46 (s, 3H), 3.25 (br d, J=12.8 Hz, 1H), 3.17 (br d, J=4.0 Hz, 1H), 2.11-1.94 (m, 2H), 1.85-1.78 (m, 5H), 1.76-1.69 (m, 2H), 1.61-1.53 (m, 3H), 1.50 (s, 3H), 1.39 (br s, 5H), 1.30-1.26 (m, 4H), 1.19 (s, 3H), 1.04 (d, J=6.4 Hz, 6H), 0.93 (s, 3H) .

### Example 24

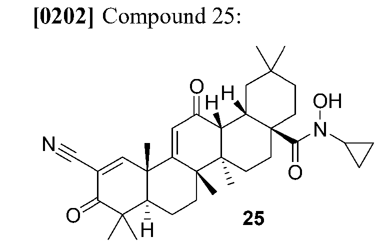

### Synthesis route:

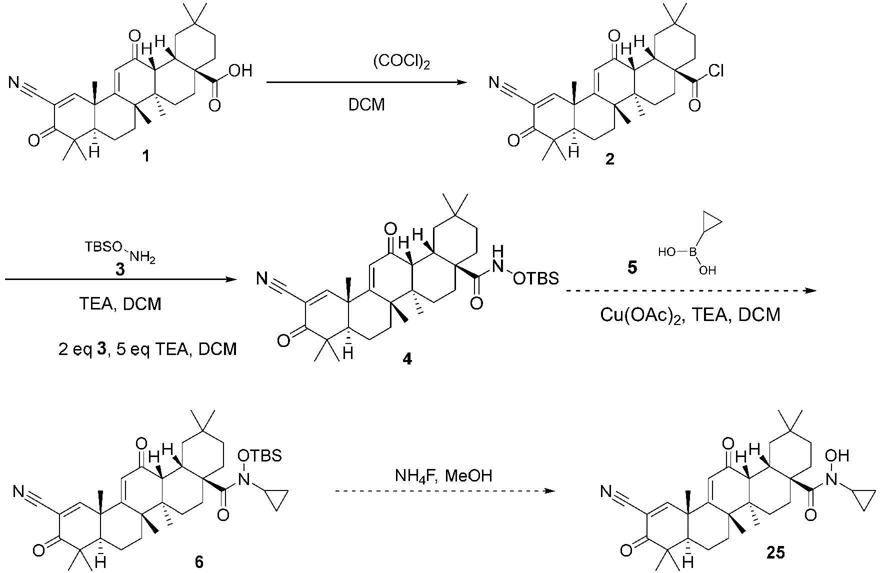

### Example 25

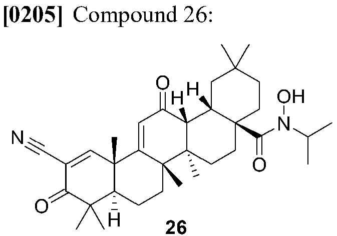

### Synthesis route:

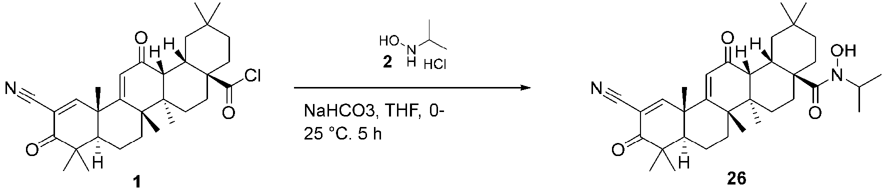

### Step 1: Synthesis of compound 26

Anhydrous tetrahydrofuran (2 mL) and sodium bicarbonate (204.87 mg, 2.44 mmol, 94.89 µL, 4 eq) were added sequentially to the material **2** (204.07 mg, 1.83 mmol, 3 eq). The reaction solution was then cooled in an ice-water bath. The material 1 (311 mg, 609.68 µmol, 1 eq) was dissolved in anhydrous tetrahydrofuran (3 mL) and then slowly added dropwise to the stirred reaction solution. The reaction solution was slowly heated to room temperature and stirred for 5 h. LCMS monitoring showed that the raw material reacted completely. The reaction solution was filtered, and the filter cake was washed with dichloromethane (20 mL*3). The filtrate was concentrated and subjected to plate separation (preparative thin-layer chromatography, petroleum ether/ethyl acetate=2:1) to obtain the crude product. The crude product was then subjected to reverse phase preparation and lyophilized to obtain compound **26** (13.6 mg, 24.63 µmol, 4.04% yield) which was as a grayish-white solid. **Prep-HPLC:** column: Phenomenex Luna C18 150*25mm*10um; mobile phase: [H2O(0.225% FA)-ACN]; gradient: 55%-85% B over 10.0 min. **LCMS:** rt=0.669 min, 549.3 [M+H]⁺. **HPLC: rt=3.810 min. ¹H NMR** (400 MHz, DMSO-d6) δ=9.06 (s, 1H), 8.65 (s, 1H), 6.17 (s, 1H), 4.60 (quin, *J*=6.6 Hz, 1H), 3.19 (br d, *J*=3.2 Hz, 1H), 3.10-2.98 (m, 1H), 2.23-2.09 (m, 1H), 1.89-1.74 (m, 4H), 1.73-1.56 (m, 4H), 1.48-1.36 (m, 5H), 1.32-1.22 (m, 5H), 1.17 (s, 3H), 1.15-1.12 (m, 1H), 1.11-1.02 (m, 10H), 0.94 (s, 3H), 0.90 (s, 3H), 0.85 (s, 3H).

### Example 26

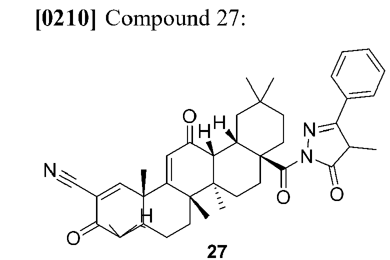

### Synthesis route:

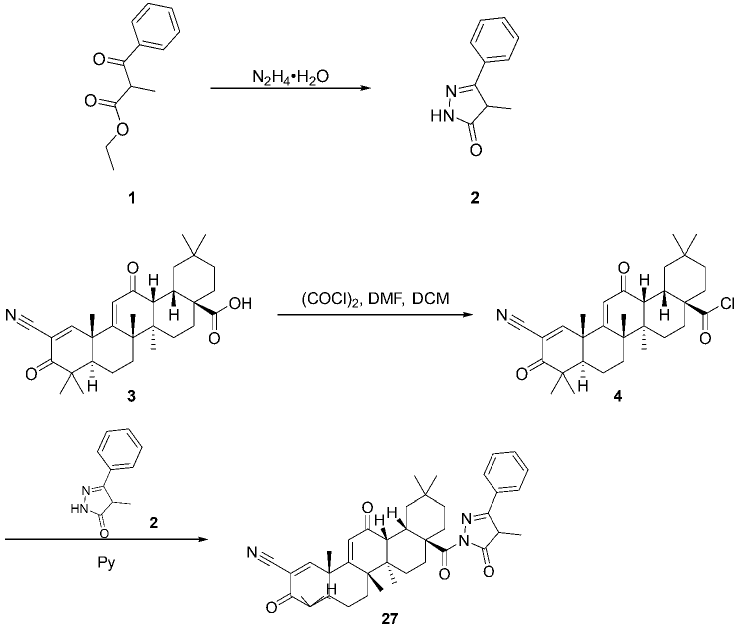

### Example 27

Compound 28:

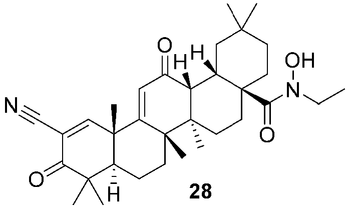

### Synthesis route:

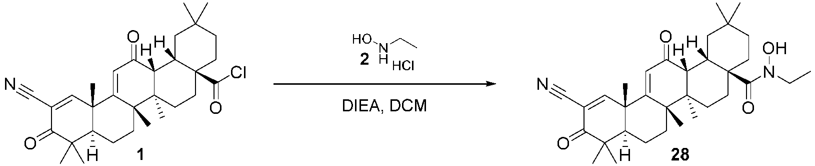

### Step 1: Synthesis of compound 28

N,N-dimethylethylamine (126.68 mg, 980.19 µmol, 170.73 µL, 10 eq) was added to the dichloromethane (0.5 mL) solution of material 2 (28.68 mg, 294.06 µmol, 3 eq, HCl), and then the dichloromethane (0.5 mL) solution of the material 1 (50 mg, 98.02 µmol, 1 eq) was added at 0°C. The reaction solution reacted at 25°C for 1 h. LCMS monitoring showed that the reaction raw material completely disappeared and the main peak was the target product. The reaction solution was concentrated and subjected to large plate separation (preparative thin-layer chromatography, petroleum ether:ethyl acetate=1:1) to obtain a crude product. The crude product was isolated and purified by reverse-phase preparation [water (formic acid)-acetonitrile system] to obtain the compound **28** (4.0 mg, 7.27 µmol, yield 7.42%, purity 97.25%) which was a white solid. Prep-HPLC: column: Phenomenex Luna C18 150*25mm*10um; mobile phase: [H₂O (0.225% FA)-ACN]; gradient: 50%-80% B over 10.0 min **LCMS:** rt=0. 671 min, 535.4 [M+H]⁺. **HPLC:** rt=2.253 min. **¹H NMR** (400 MHz, DMSO-d6) δ=9.50 (s, 1H), 8.65 (s, 1H), 6.18 (s, 1H), 3.66-3.54 (m, 1H), 3.51-3.40 (m, 1H), 3.18 (br s, 1H), 3.11-2.98 (m, 1H), 2.16-2.07 (m, 1H), 1.87-1.74 (m, 4H), 1.71-1.58 (m, 4H), 1.48-1.37 (m, 5H), 1.34-1.25 (m, 2H), 1.24 (s, 3H), 1.17 (s, 3H), 1.13-1.04 (m, 8H), 0.94 (s, 3H), 0.90 (s, 3H), 0.85 (s, 3H).

### Example 28

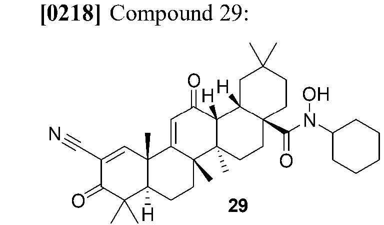

### Synthesis route:

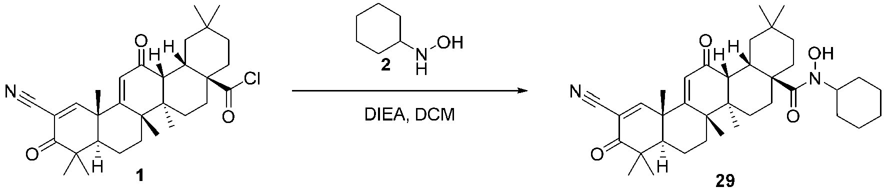

### Step 1: Synthesis of compound 29

N,N-dimethylethylamine (380.04 mg, 2.94 mmol, 512.18 µL, 5 eq) was added to the dichloromethane (5 mL) solution of the material **2** (267.53 mg, 1.76 mmol, 3 eq, HCl). Then the dichloromethane (2 mL) solution of the material **1** (300 mg, 588.11 µmol, 1 eq) was added at 0°C. The reaction solution reacted at 25°C for 1 h. LCMS monitoring showed that the reaction raw material completely disappeared and the main peak was the target product. The reaction solution was concentrated and subjected to prep-TLC (petroleum ether:ethyl acetate=1:1) to obtain a crude product. The crude product was isolated and purified by reverse-phase preparation [water (formic acid)-acetonitrile system] to obtain the compound **29** (10.70 mg, 18.00 µmol, yield 3.06%, purity 99.04%) which was a white solid. Prep-HPLC: column: Phenomenex Luna C18 150*25mm*10um; mobile phase: [H₂O (0.225% FA)-ACN]; gradient: 60%-90% B over 10.0 min. **LCMS:** rt=0.703 min, 589.4 [M+H]⁺. **HPLC:** rt=2.583 min. **¹H NMR** (400 MHz, DMSO-d6) δ=9.09 (s, 1H), 8.65 (s, 1H), 6.17 (s, 1H), 4.27-4.14 (m, 1H), 3.19 (br d, *J*=3.8 Hz, 1H), 3.11-2.96 (m, 1H), 2.23-2.07 (m, 1H), 1.83 (br s, 3H), 1.78-1.63 (m, 6H), 1.60-1.48 (m, 6H), 1.48-1.40 (m, 5H), 1.40-1.35 (m, 1H), 1.31-1.21 (m, 7H), 1.17 (s, 3H), 1.13-1.08 (m, 2H), 1.06 (s, 3H), 0.94 (s, 3H), 0.89 (s, 3H), 0.85 (s, 3H).

### Example 29

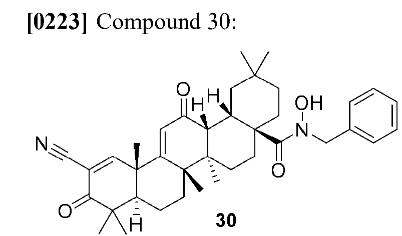

### Synthesis route:

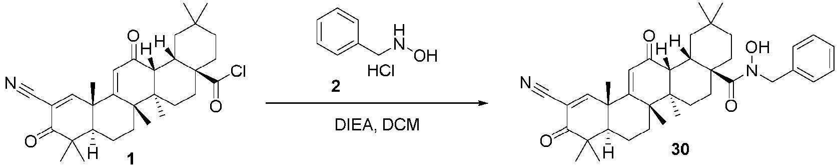

### Step 1: Synthesis of compound 30

N,N-dimethylethylamine (126.68 mg, 980.19 µmol, 170.73 µL, 5 eq) was added to the dichloromethane (2 mL) solution of the compound **2** (93.87 mg, 588.11 µmol, 3 eq, HCl), followed by adding the dichloromethane (1 mL) solution of the compound **1** (100 mg, 196.04 µmol, 1 eq) at 0°C. The reaction solution reacted at 25°C for 1 h. LCMS monitoring showed that the reaction raw material completely disappeared and the main peak was the target product. The reaction solution was concentrated and subjected to prep-TLC (petroleum ether:ethyl acetate=1:1) to obtain a crude product. The crude product was isolated and purified by reverse-phase preparation [water (formic acid)-acetonitrile system] to obtain the compound **30** (3.20 mg, 5.14 µmol, yield 2.62%, purity 95.93%) which was a white solid. **Prep-HPLC:** column: Phenomenex Luna C18 150*25mm*10µm; mobile phase: [H₂O ( 0.225% FA)-ACN]; gradient: 55%-85% B over 10.0 min. **LCMS:** rt=0. 725 min, 597.3 [M+H]⁺. **HPLC:** rt=2.419 min. **¹H NMR** (400 MHz, DMSO-d6) δ=9.80 (s, 1H), 8.64 (s, 1H), 7.35-7.22 (m, 5H), 6.18 (s, 1H), 4.84-4.58 (m, 2H), 3.16 (br s, 1H), 3.12-3.01 (m, 1H), 2.19-2.08 (m, 1H), 1.89-1.76 (m, 4H), 1.75-1.56 (m, 4H), 1.47-1.40 (m, 4H), 1.37-1.25 (m, 3H), 1.20-1.10 (m, 7H), 1.06 (s, 4H), 0.94 (s, 3H), 0.91 (s, 3H), 0.86 (s, 3H).

### Example 30

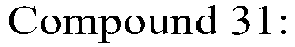

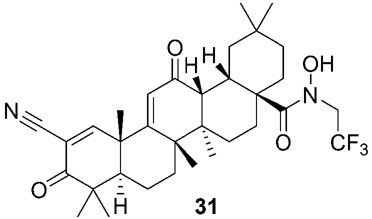

### Synthesis route:

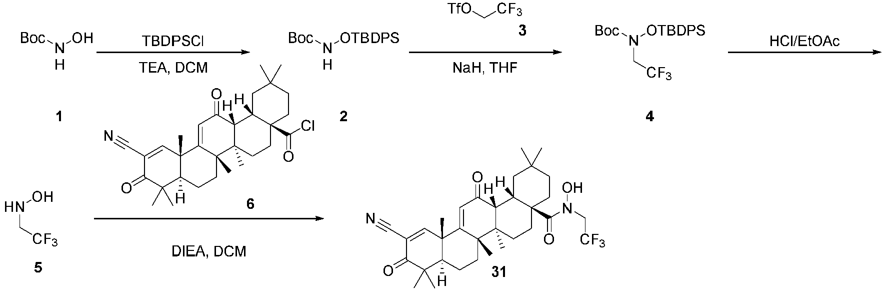

### Step 1: Synthesis of intermediate 2

The dichloromethane (20 mL) solution of material 1 (2 g, 15.02 mmol, 1 eq) was added with triethylamine (1.67 g, 16.52 mmol, 2.30 mL, 1.1 eq) and tert-butyldiphenylchlorosilane (4.13 g, 15.02 mmol, 3.84 mL, 1 eq). The reaction solution reacted at 25°C for 12 h. LCMS monitoring showed that the reaction raw material completely disappeared and the main peak was the target product. The reaction solution was concentrated under reduced pressure, and the resulted crude product was purified by normal-phase silica gel column chromatography (petroleum ether:ethyl acetate=1:0 to 100:1), and then concentrated under reduced pressure to obtain the intermediate 2 (5.5 g, 14.06 mmol, yield 93.62%, purity 95%) which was colorless oily. LCMS: Rt=0.711 min, 394.2 [M+Na]⁺ESI ¹H NMR (400 MHz, CHLOROFORM-d) δ=7.80-7.71 (m, 4H), 7.49-7.37 (m, 6H), 6.71 (s, 1H), 1.38 (s, 9H), 1.15 (s, 9H).

### Step 2: Synthesis of intermediate 4

Sodium hydride (322.98 mg, 8.07 mmol, purity 60%, 1.5 eq) was slowly added to the tetrahydrofuran (30 mL) solution of the intermediate **2** (2 g, 5.38 mmol, 1 eq) at 0°C. The reaction solution reacted at 0°C for 30 min, then slowly added with the material **3** (3.12 g, 13.46 mmol, 2.5 eq). The reaction solution reacted at 25°C for 12 h. LCMS monitoring showed that the reaction raw material completely disappeared and the main peak was the target product. The reaction solution was slowly poured into a saturated ammonium chloride aqueous solution (20 mL), and extracted with ethyl acetate (20 mL*2). The combined organic phase was washed with saturated brine (20 mL), dried over anhydrous sodium sulfate, and concentrated under reduced pressure, and the crude product obtained was purified by normal-phase silica gel column chromatography (petroleum ether:ethyl acetate=1:0) and then concentrated under reduced pressure to obtain the intermediate **4** ( 1.87 g, 3.71 mmol, yield 68.93% , purity 90%) which was colorless oily. **LCMS:** Rt=0.801 min, 476.2 [M+Na ] + . ESI **¹H NMR** (400 MHz, CHLOROFORM-d) δ=7.74-7.69 (m, 4H), 7.48-7.43 (m, 2H), 7.41-7.37 (m, 4H), 3.93-3.82 (m, 2H), 1.26 (s, 9H), 1.17 (s, 9H).

### Step 3: Synthesis of intermediate 5

An ethyl acetate hydrochloride solution (2 M, 5.00 mL, 9.07 eq) was added to the intermediate **4** (500 mg, 1.10 mmol, 1 eq), and the reaction solution reacted at 25°C for 12 h. TLC monitoring showed complete disappearance of the reaction raw material. The reaction solution was concentrated under reduced pressure, slurried with petroleum ether (2 mL) and filtered to obtain the concentrated filter cake to obtain the intermediate **5** (50 mg, 330.00 µmol, 29.94% yield, HCl) which was a white solid. **¹H NMR** (400 MHz, DMSO-d6) δ=3.94-3.81 (m, 2H).

### Step 4: Synthesis of compound 31

N,N-dimethylethylamine (63.34 mg, 490.09 µmol, 85.36 µL, 5 eq) was added to the dichloromethane (0.5 mL) solution of the intermediate **5** (44.55 mg, 294.06 µmol, 3 eq, HCl), followed by adding the dichloromethane (0.5 mL) solution of the material **6** (50 mg, 98.02 µmol, 1 eq) at 0°C. The reaction solution reacted at 25°C for 1 h. LCMS monitoring showed that the reaction raw material completely disappeared and the main peak was the target product. The reaction solution was concentrated and subjected to prep-TLC (petroleum ether: ethyl acetate=2: 1) to obtain a crude product. The crude product was isolated and purified by reverse-phase preparation [water (formic acid)-acetonitrile system] to obtain the compound **31** (7.48 mg, 12.58 µmol, yield 12.84%, purity 99.04%) which was a white solid. **Prep-HPLC:** column: Phenomenex Luna C18 150*25mm*10um; mobile phase: [H₂O (0.225% FA)-ACN]; gradient: 53%-83% B over 10.0 min. **LCMS:** rt=0. 661 min, 589.4 [M+H]⁺. **HPLC:** rt=2.969 min. **¹H NMR** (400 MHz, DMSO-d6) δ=10.20 (s, 1H), 8.65 (s, 1H), 6.19 (s, 1H), 4.63-4.44 (m, 1H), 4.28-4.10 (m, 1H), 3.14-2.97 (m, 2H), 2.19-2.07 (m, 1H), 1.93-1.76 (m, 4H), 1.74-1.59 (m, 4H), 1.46-1.41 ( m , 4H), 1.40-1.26 (m, 3H), 1.22 (s, 3H), 1.19-1.10 (m, 5H), 1.06 (s, 3H), 0.95 (s, 3H), 0.90 (s, 3H), 0.86 (s, 3H).

### Example 31

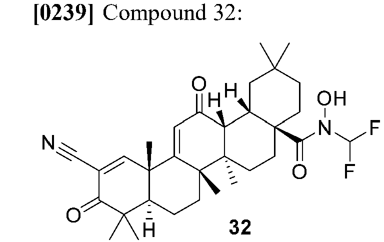

### Synthesis route:

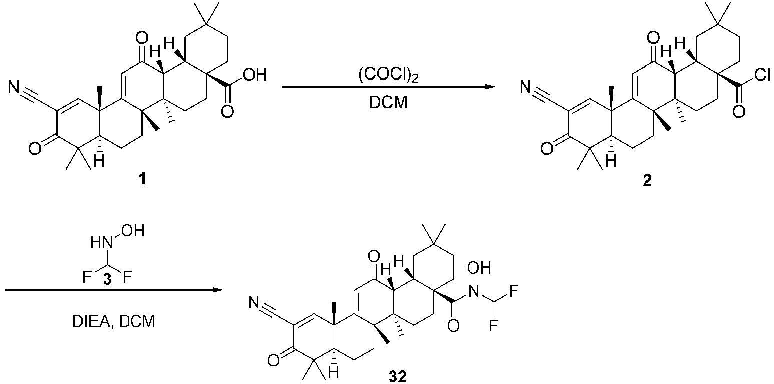

### Example 32

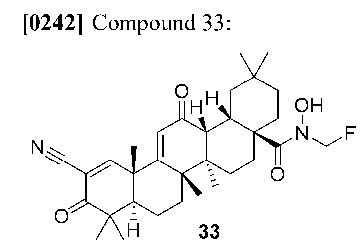

### Synthesis route:

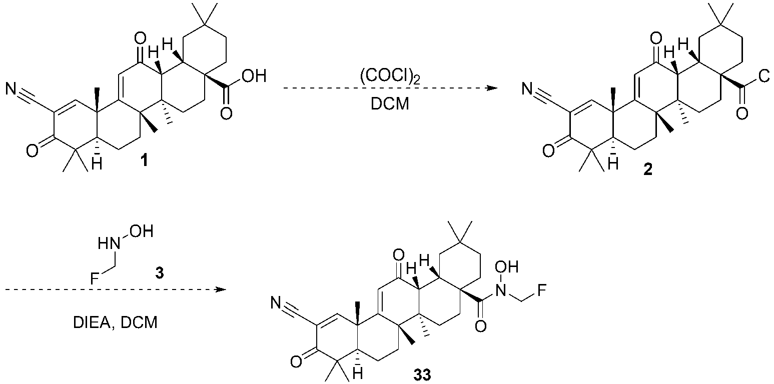

### Example 33

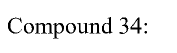

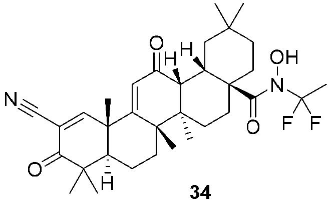

### Synthesis route:

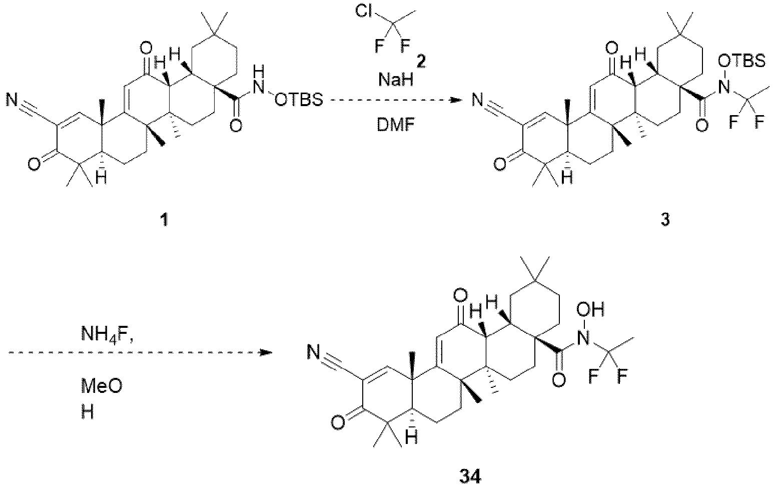

### Example 34

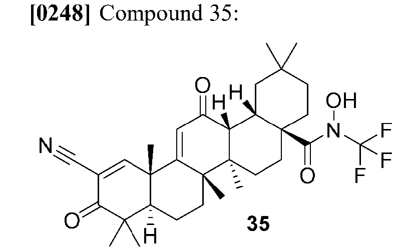

### Synthesis route:

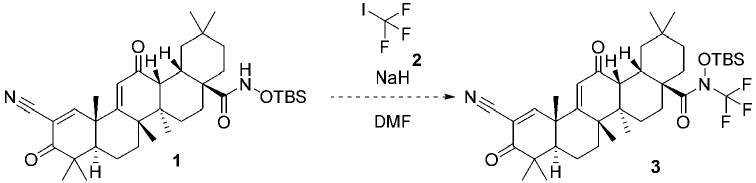

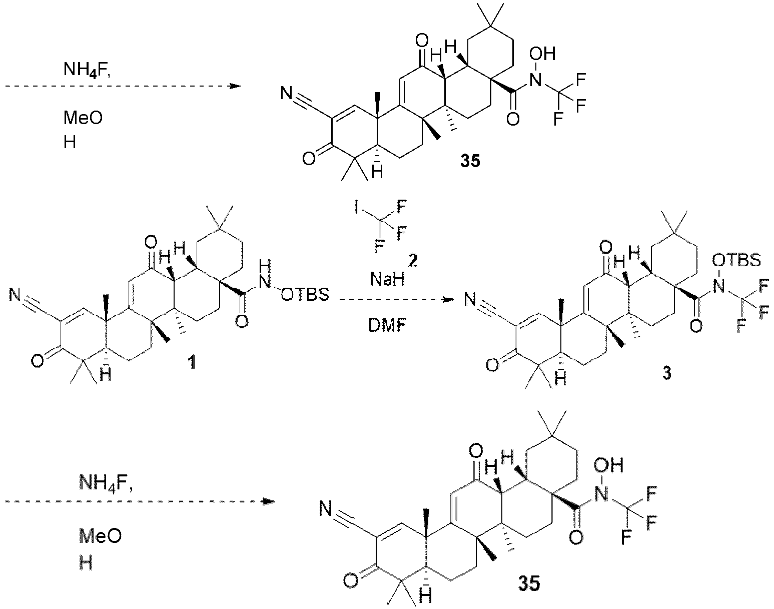

### Example 35

### Biological evaluation

### Experimental Example 1: In vitro human Nrf2 receptor agonist activity assay

Reagents: Opti-MEM serum-free medium; PEI 40000 transfection reagent; DMEM complete medium.

Equipment: Cx7Pro high content rapid imaging platform, etc.

For each well of cells, 10 µL of Opti-MEM serum-free medium (Cienry) was used to dilute 360 ng of Nrf2 phase transition probe plasmid, mixed thoroughly to form a DNA dilution, and allowed to stand for 5 minutes. 10 µL of Opti-MEM serum-free medium was used to dilute 0.6 µL of PEI 40000 transfection reagent (YEASEN), mixed thoroughly to form a PEI 40000 dilution, and allowed to stand for 5 minutes. The DNA dilution and PEI40000 dilution were mixed and incubated at room temperature for 20 minutes to form DNA-PEI cationic nucleic acid transfection reagent complexes. 20 µL of cell growth medium was removed from each well, and 20 µL of DNA-PEI cationic nucleic acid transfection reagent complex was added to each well. The culture plate was shaken to mix gently. Cells were cultured in an incubator at 37°C with 5% CO₂. 1 hour after transfection, 75 µL of cell growth medium was removed from each well, and 75 µL of fresh pre-warmed DMEM complete medium (Meilunbio) was added to each well, maintaining a total volume of 100 µL per well. 18 hours after transfection, serially diluted drugs (0.0625 nM, 0.125 nM, 0.25 nM, 0.5 nM, 1 nM, 2 nM, 4 nM, 8 nM, 16 nM, 32 nM, 128 nM) were first mixed into the pre-warmed DMEM complete medium. 100 µL of medium containing the serially diluted drugs was added to each sample well, resulting in a final volume of 200 µL per well. 6 hours after drug addition, 16 fields of view were randomly selected for photographing for each well using a Cx7Pro high-content rapid imaging platform (Thermo Fisher) with a 20x high-resolution lens. The nucleus location was determined by the independently expressed NLS-mTagBFP2 in the probe, thus determining the number of transfected cells. The total fluorescence intensity of the Nrf2 phase transition probe was calculated. The total fluorescence intensity of the phase transition "droplet" of Nrf2 phase transition probe in each cell was statistically analyzed. The total fluorescence intensity of the Nrf2 phase transition probe in each cell of the drug group was compared with the total fluorescence intensity of the Nrf2 phase transition probe in each cell of the DMSO group. The EC₅₀ of each drug was statistically analyzed using the log(agonist) vs. response - Variable slope (four parameters) analysis method in GraphPad Prism. The results are as shown in Table 1.

**Table 1. Results of in vitro human Nrf2 receptor agonist activity assays of representative compounds of the present application**

| Compound | human Nrf2 receptor agonist activity EC₅₀ (nM) |
|---|---|
| Edaravone | Inactive |
| Omaveloxolone | 6.60 |
| 1 | 5.41 |
| 3 | 21.96 |
| 4 | 0.65 |
| 5 | 4.58 |
| 11 | 5.48 |
| 13 | 51.10 |
| 14 | 55.70 |
| 16 | 2.05 |
| 17 | 8.27 |
| 19 | 1.67 |
| 21 | Greater than 1000 |
| 22 | Greater than 1000 |
| 24 | 1.57 |
| 26 | 3.01 |
| 28 | 1.91 |
| 29 | 7.58 |
| 30 | 7.97 |
| 31 | 4.10 |

Biological experimental data are as shown in Table 1. Furthermore, test data show that multiple molecules, especially the compounds 1, 4, 5, 11, 16, 17, 19, 24, 26, 28, 29, 30, and 31 prepared in the present application all exhibit single-digit nanomolar human Nrf2 receptor agonist activity, significantly superior to the control drug Omaveloxolone. The above *in vitro* human Nrf2 receptor agonist activity data indicate that the compounds of the present application, their pharmaceutically acceptable salts, and stereoisomers can be used for the preparation of NRF2 activators.

Furthermore, comparative experiments with the control drug Omaveloxolone showed that both the compounds of the present application and the control drug Omaveloxolone have human Nrf 2 receptor agonist activity, but there are still significant differences in the activity of the compounds in terms of DPPH free radical scavenging and MDA anti-lipid peroxidation, as shown in Experiment Examples 2-4.

### Experimental Example 2: DPPH Free Radical Scavenging Capacity Test

Experimental objective: To determine the DPPH free radical scavenging capacity of the compounds of the present application.

### Experimental materials:

| **Material** | **Manufacturer** | **Catalog No.** |
|---|---|---|
| Dimethyl sulfoxide | Sigma | D2650 |
| D4Plus pipette tip rack | Tecan | 30097371 |
| T8Plus pipette tip rack | Tecan | 30097370 |
| 96-well plate | Corning | 3599 |
| Anhydrous ethanol | Sangon | A500737 |
| 2,2-Diphenyl-1-picrylhydrazyl (DPPH) | MCE | HY-112053 |

### Experimental instruments:

| **Instrument** | **Manufacturer** | **Model** |
|---|---|---|
| Digital Dispenser | Tecan | HP D300 |
| Microplate Reader | FlexStation 3 | Molecular Devices |
| Centrifuge | Eppendorf | 5425R |
| Tissue Grinder | Shanghai Jingxin | JXFSTPRP-CL-48 |
| Dry Thermostat | Hm-Kylin | GL-150B |

### Experimental method:

First, 20 µL of the test compound was added to a 96-well plate and serially diluted at a 1:2 ratio with DMSO. Then, 200 µL of 200 µM DPPH prepared in anhydrous ethanol as solvent was added to each well. After gentle shaking, incubation was performed at room temperature in the dark for 30 minutes. Absorbance value at 517 nm was measured using a microplate reader. The DPPH scavenging rate was calculated using the following formula: DPPH scavenging rate% = (1-Ai/A0)* 100%, where Ai was the absorbance value of the sample and A0 was the absorbance value of the DMSO control group. Data were processed using XLfit 5.3.1.3 software, and IC₅₀ values were obtained using a non-linear fitting formula. Results are shown in Table 2.

**Table 2: Test results of DPPH free radical scavenging capacity of representative compounds of the present application**

| Compound | Free radical scavenging DPPH EC₅₀ (µM) |
|---|---|
| Edaravone | 73.28 µM |
| Omaveloxolone | Inactive |
| 19 | 182.88 |
| 24 | 239.81 |

The results show that compounds 19 and 24 prepared in the present application possess DPPH free radical scavenging capacity, while Omaveloxolone has no such capacity.

### Experimental Example 3: MDA Anti-lipid peroxidation Capacity Test

Experimental objective: To test the MDA anti-lipid peroxidation capacity of the compounds of the present application.

### Experimental materials:

| **Material** | **Manufacturer** | **Catalog No.** |
|---|---|---|
| DPBS (1×) | Corning | 21-031-CVC |
| Dimethyl sulfoxide | Sigma | D2650 |
| D4Plus pipette tip rack | Tecan | 30097371 |
| T8Plus pipette tip rack | Tecan | 30097370 |
| 96-well plate | Corning | 3599 |
| Ascorbic acid | ST1434 | Beyotime |
| Lipid Peroxidation (MDA) Assay Kit | S0131M | Beyotime |

### Experimental instruments:

| **Instrument** | **Manufacturer** | **Model** |
|---|---|---|
| Digital Dispenser | Tecan | HP D300 |
| Microplate Reader | FlexStation 3 | Molecular Devices |
| Centrifuge | Eppendorf | 5425R |
| Tissue Grinder | Shanghai Jingxin | JXFSTPRP-CL-48 |
| Dry Thermostat | Hm-Kylin | GL-150B |

### Experimental method:

First, brain tissue homogenate was extracted: an adult male SD rat was anesthetized with isoflurane and sacrificed by cervical dislocation. The whole brain was removed, and washed twice with DPBS, and the meninges were stripped. The brain was transferred to a 50 mL centrifuge tube containing 10 mL of DPBS, cut into pieces with scissors, and dispensed into 10 1.5 mL centrifuge tubes. Three grinding beads were added to each tube, and the tissue was ground at 90 Hz for 60 min, repeated 3 times. The ground tissue homogenate was transferred to a new 50 mL centrifuge tube, and DPBS was added to a total volume of 30 mL and thoroughly mixed. Then, 20 µL of test compound was added to a 96-well plate and serially diluted at a ratio of 1:3 with DMSO. Subsequently, 100 µL of brain tissue homogenate, 50 µL of DPBS, and 50 µL of 200 µg/mL vitamin C were added sequentially, and a series of concentration standards were prepared as a standard curve. After shaking, the plate was incubated at 37°C for 1 hour. Then, 400 µL of MDA working solution was added, and the plate was heated at 100°C for 15 minutes. After cooling to room temperature, the plate was centrifuged at 1000 g for 10 minutes. 200 µL of supernatant was transferred to a new plate, and absorbance value at 532 nm was measured using a microplate reader. The MDA clearance rate was calculated using the following formula: MDA clearance rate% = [((A1-A0)-(A2-A0))/((A1-A0)- (A3-A0))]*100%, where A1 was the absorbance value of the high control group, A2 was the absorbance value of the sample, A3 was the absorbance value of the low control group, and A0 was the absorbance value of the blank group. Data were processed using XLfit 5.3.1.3 software, and IC₅₀ values were obtained using a non-linear fitting formula. Results are shown in Table 3.

**Table 3. Test results of MDA anti-lipid peroxidation capacity of representative compounds of the present application**

| Compound | Antioxidant MDA EC₅₀ (µM) |
|---|---|
| Edaravone | 55.07 |
| Omaveloxolone | Inactive |
| 13 | 225.96 |
| 14 | 212.23 |
| 19 | 5.01 |
| 24 | 6.71 |
| 26 | 34.78 |
| 28 | 33.95 |

The results show that Compounds 19, 24, 26, and 28 prepared in the present application possess anti-lipid peroxidation capacity, and the activity is superior to Edaravone, while Omaveloxolone has no such activity.

### Experimental Example 4 Interventional effect of representative compounds on ferroptosis process (qPCR)

**1) Experimental Objective:** The interventional effect of a series of compounds on the ferroptosis process was tested by detecting changes in the expression levels of key genes in the ferroptosis signaling pathway at the cellular level. The specific indicator was the mRNA level of PTGS2, a key gene in ferroptosis process (detected by real-time fluorescent quantitative RT-PCR)

### 2) Experimental methods:

HT-1080 cells (Cat. No. CCL-121, Shanghai Cell Bank, originally from ATCC) were plated in 6-well plates at 4×10⁵ cells/well for 22 h. HT-1080 cells were treated in advance with different concentration gradients of compounds (1.37 nM to 333 nM, 6 concentration gradients in total) for 1 hour. Then, HT-1080 cells were treated with 200 nM classical ferroptosis inducer RSL3 (Cat. No. HY-100218A, MCE, USA) for 16 hours. mRNA was extracted, reverse transcribed to cDNA, and PTGS2 mRNA levels were detected by qRT-PCR (model: ABI7500, Thermo Fisher, USA). Ferroptosis inhibitor Fer-1 (Cat. No. HY-100579, MCE, USA) was used as a positive control.

### 3) Experimental results:

The log(agonist) vs. response-Variable slope (four parameters) analysis method in GraphPad Prism was used to calculate the IC₅₀ of each drug. Results are shown in Table 4.

**Table 4: Intervention effect of compounds of the present application on the ferroptosis process**

| RSL3 Treatment and qPCR Detection of PTGS2 mRNA | |
|---|---|
| Compound | PTSG2 IC₅₀ (nM) |
| Omaveloxolone | Inactive |
| 26 | 105.8 |
| 28 | 154.9 |

The results show that representative compounds 26 and 28 prepared in the present application both can inhibit ferroptosis, while Omaveloxolone has no such activity.

The above pharmacological experimental demonstrates that the preferred NRF2-Keap1 compounds prepared in the present application, such as 19, 24, 26, 28, etc., not only maintain Nrf2 agonist activity similar to the marketed Nrf2 agonist Omaveloxolone but also possess functions of scavenging DPPH free radicals, inhibiting the production of lipid peroxide MDA, or intervening in ferroptosis.

Meanwhile, the present application verifies through *in vivo* biological experiment that the compounds of the present application have significant effects on various diseases, the related diseases including: cerebral small vessel disease, mitochondrial encephalomyopathy, autism spectrum disorder, Rett syndrome, Friedreich ataxia, stroke, hemorrhagic stroke, ischemic stroke, multiple sclerosis, amyotrophic lateral sclerosis, schizophrenia, cognitive impairment in schizophrenia, Parkinson's disease, cognitive impairment in Parkinson's disease, Alzheimer's disease, vascular dementia, epilepsy, Huntington's chorea, heart failure, myocardial infarction, renal failure, and renal ischemia. In particular, they show significant effects on diseases such as stroke, multiple sclerosis, and amyotrophic lateral sclerosis, and can be used in the preparation of a medicament for preventing or treating stroke, multiple sclerosis, and amyotrophic lateral sclerosis.

### Experimental Example 5 Tissue distribution experiment of representative compounds in SD rats

Test animals: SD rats, male, weighing approximately 250±20 g, randomly divided into groups, each group having 3 rats.

Vehicle: DMSO: 10% solutol (5%: 95%).

### Experimental method:

The drugs were administered via gavage, with the dosage of 20 mg/kg (30 mg/kg for compound 30). 12 h before administration, the animals were fasted, but had free access to water. Plasma was collected at specific time points after administration, and brain tissue was collected after cardiac perfusion. After pretreatment, the samples were analyzed by LC-MS/MS to determine the concentrations of the analytes in plasma and brain. The experimental results are shown in Table 4.

**Table 4: Concentrations in plasma and brain of SD rats after administration**

| | | Brain (ng/g) | | | Plasma (ng/mL) | | |
|---|---|---|---|---|---|---|---|
| | Time point | 0.25 h | 1 h | 6h | 0.25 h | 1 h | 6h |
| 19 | Mean±SD | 4.54±3.47 | 3.57±2.28 | 1.82±1.54 | 94.2±63.7 | 164±33 | 68.8±15.6 |
| | B/P | 0.06 | 0.02 | 0.02 | / | / | / |
| 24 | Mean±SD | 161±166 | 88.1±57.4 | 75.7±65.8 | 111±57.5 | 316±194 | 324±204 |
| | B/P | 1.90 | 0.39 | 0.21 | / | / | / |
| 31 | Mean±SD | 6.93±1.19 | 20.5±10.1 | 80.9±22.9 | N/A | N/A | N/A |
| | B/P | / | / | / | / | / | / |
| | Time point | 0.5 h | 2h | 8 h | 0.5 h | 2 h | 8 h |
| 24 | Mean±SD | 9.96±7.22 | 6.01±0.52 | 56.9±38.8 | 146±40.8 | 288±196 | 254±50.2 |
| | B/P | 0.06 | 0.03 | 0.23 | / | / | / |
| 26 | Mean±SD | 146±153 | 109±111 | 637±465 | 113±104 | 103±99.2 | 383±138 |
| | B/P | 2.89 | 2.10 | 1.94 | / | / | / |

### Experimental Example 6 Protective effect of representative compounds against ischemic stroke

**1) Reagents:** Omaveloxolone, MedChemexpress Biotechnology Co., USA; Transient Middle Cerebral Artery Occlusion Model (tMCAO) suture, Beijing Ruiwode Life Science Technology Co., Ltd.; DMAO, Beyotime Biotechnology Co., Ltd.; Solutal, Sigma-Aldrich (Shanghai) Trading Co., Ltd.; Physiological saline, Sinopharm Chemical Reagent Co., Ltd.; TTC (2,3,5-Triphenyltetrazolium chloride), Sigma-Aldrich (Shanghai) Trading Co., Ltd.

### 2) Establishment of cerebral ischemia rat model using tMCAO method:

Rats were anesthetized with 10% chloral hydrate (350 mg/kg) and fixed in a supine position on the operating table. The neck was disinfected. A midline neck incision was made, and the intermuscular space between the left sternocleidomastoid muscle and the sternohyoid muscle was bluntly dissected to expose the common carotid artery. The common carotid artery was picked out with ophthalmic curved forceps and threaded with silk suture for later use. The external carotid artery was dissected under the right digastric muscle and hyoid bone and threaded with silk suture for later use. The suture was passed through and tied tightly around the common carotid artery. A small incision was made at the free end of the common carotid artery and a loose knot was tied. The occluder was inserted through the incision along the common carotid artery. The occluder was slowly advanced and stopped when resistance was felt. The loose knot tied in advance was tied tightly. The body temperature was maintained at 37°C during the operation. The occluder was removed after 1.5 h. For the rats in the sham group, the occluder was not inserted after the blood vessel was dissected, but the other operation steps were the same.

### 3) Experimental grouping and administration:

SD male rats, weighing 230-250 g, 48 rats, were randomly divided into the sham group, model group, Omaveloxolone 3 mg/kg group, Omaveloxolone 9 mg/kg group, compound 19 2.74 mg/kg group, and compound 28 2.89 mg/kg group. After 3 days of acclimatization, the rats were administered via tail vein injection during the model establishment and occluder insertion. Rats in the sham group and the model group were given an equal volume of vehicle (10% DMSO + 10% solutal + 80% physiological saline).

### 4) Indicator detection:

**mNSS score:** Neurological behavior scores were assessed 24 hours after model establishment. The evaluation mainly focused on sensory and motor aspects to assess the neurological function of ischemic stroke rats.

**Cerebral infarction area measurement:** Rats were euthanized with an overdose of chloral hydrate. The brains were removed and placed in a -20°C refrigerator for 20 min. The brains were taken out and placed in a brain matrix for sectioning into 2 mm thick slices. The brain slices were placed in 2% TTC solution, protected from light, and incubated in a 37°C incubator for 15 min. After staining, images were taken for cerebral infarction area analysis.

### 5) Statistical methods:

All data in this study were expressed as mean±standard deviation (Mean±SD) and analyzed using GraphPad Prism 7.0. Differences among multiple groups were evaluated by one-way ANOVAs followed by Tukey's test. Behavioral tests were analyzed using the Kruskal-Wallis test method. P < 0.05 was considered statistically significant.

### 6) Experimental results:

### Effects of compounds 19 and 28 on neurological function in ischemic stroke rats:

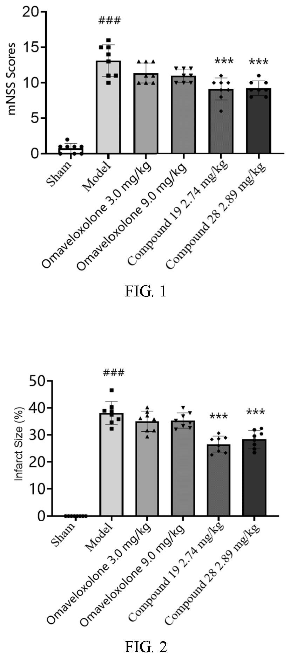

As shown in FIG. 1, Omaveloxolone at 3 mg/kg and 9 mg/kg had no effect on the mNSS score in cerebral ischemia rats. At the same molar dose as Omaveloxolone 3 mg/kg, compound 19 (2.74 mg/kg) and compound 28 (2.89 mg/kg) both significantly reduced the mNSS score in cerebral ischemia rats.

### Effects of compounds 19 and 28 on cerebral infarction area in ischemic stroke rats:

As shown in FIG. 2, Omaveloxolone at 3 mg/kg and 9 mg/kg had no effect on the infarction area in cerebral ischemia rats. At the same molar dose as Omaveloxolone 3 mg/kg, compound 19 (2.74 mg/kg) and compound 28 (2.89 mg/kg) both significantly reduced the infarction area in cerebral ischemia rats.

**7) Conclusion:** The novel Nrf2-Keap1 uncoupling agents involved in the present application, such as compounds 19 and 28, can reduce the cerebral infarction area in ischemic stroke rats and have a protective effect against neurological damage.

### Experimental Example 7 Effect of representative compounds on neurobehavioral function in multiple sclerosis model mice

### 1. Materials and Methods

### 1) Main reagents

| **Material** | **Manufacturer** | **Country** |
|---|---|---|
| MOG₃₅₋₅₅ peptide | Sangon | China |
| Pertussis toxin | Aibixin | China |
| Complete Freund's Adjuvant, 5 mg/ml | chondrex | USA |
| Solutol^{®}HS 15 | Sigma-Aldrich | Germany |
| Tween-80 | Beyotime | China |
| DMSO | Beyotime | China |
| PEG300 | Sigma-Aldrich | Germany |

### 2) Experimental animals and grouping and administration

Female C57BL/6J mice were randomly divided into 5 groups: sham group, model group, Omaveloxolone 5 mg/kg group, Omaveloxolone 15 mg/kg group, and compound 24 4.69 mg/kg, with 8 mice in each group. The drugs were administered by gavage. Mice in the sham group and the model group were given a volume of vehicle (10% solutol + 90% physiological saline). Each group was administered twice daily for 42 consecutive days

### 3) Preparation of multiple sclerosis model - EAE model

A mixed emulsion containing myelin oligodendrocyte glycoprotein MOG35-55 peptide and complete Freund's adjuvant (CFA) containing *Mycobacterium tuberculosis* was injected subcutaneously into the back of the mice. Pertussis toxin (PTX) was injected intraperitoneally on the day of immunization and 48 hours later to establish the EAE (experimental autoimmune encephalomyelitis) model.

### 4) Body weight and neurological function scoring (5-point scale)

From the day of immunization induction (Day 0), mice were weighed and subjected to neurological function scoring daily. Neurological function scoring criteria (5-point scale): 0, no clinical deficits; 1, partial tail paralysis; 2, complete tail paralysis; 3, partial hind limb paralysis; 4, complete hind limb paralysis; 5, forelimb paralysis; 6, death.

### 5) Statistical treatment

All data in this study were expressed as mean±standard deviation (Mean±SD). Differences in neurological function scores and body weight between groups were compared using two-way ANOVA and Tukey's multiple comparison test. All data were analyzed using GraphPad Prism 9.0.0 software. p < 0.05 indicated statistically significant differences.

### 2. Experimental results

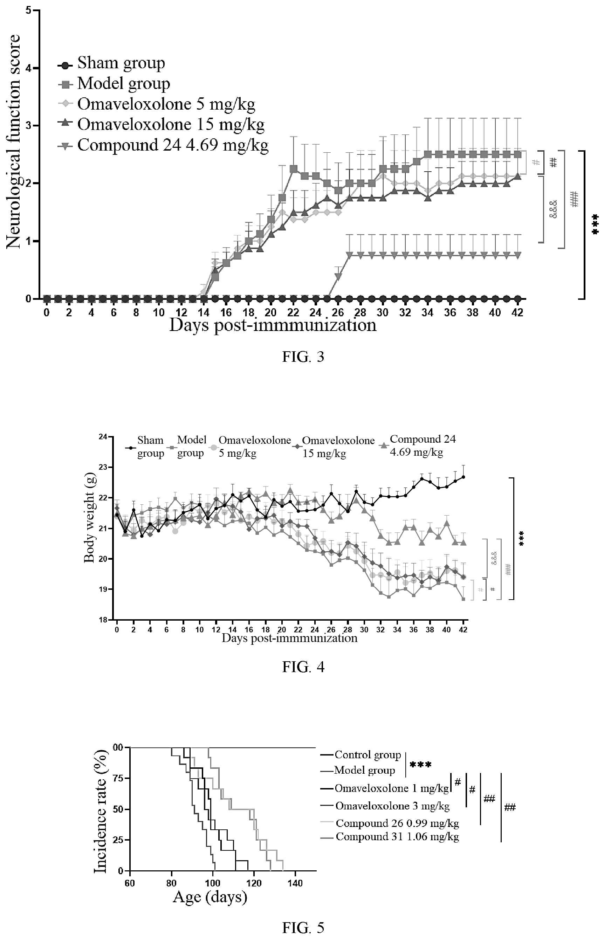

As shown in FIG. 3, Omaveloxolone 5 mg/kg and 15 mg/kg significantly reduced the neurological function scores in EAE model mice, but there was no significant difference between the two groups, indicating that Omaveloxolone 5 mg/kg reached the maximum effect. At the same molar dose as Omaveloxolone 5 mg/kg, compound 24 (4.69 mg/kg) significantly reduced the neurological function scores in EAE model mice, and the effect was superior to Omaveloxolone 15 mg/kg, with statistically significant differences.

As shown in FIG. 4, Omaveloxolone 5 mg/kg and 15 mg/kg significantly increased the body weight of EAE model mice, but there was no significant difference between the two groups, indicating that Omaveloxolone 5 mg/kg reached the maximum effect. At the same molar dose as Omaveloxolone 5 mg/kg, compound 24 (4.69 mg/kg) significantly reduced the body weight in EAE model mice, and the effect was superior to Omaveloxolone 15 mg/kg, with statistically significant differences.

### 3. Experimental conclusion

Compound 24 has a protective effect on neurological function in multiple sclerosis model mice, and its effect is superior to that of Omaveloxolone.

### Experimental Example 8 Protective Effect of representative compounds on amyotrophic lateral sclerosis model mice

### 1. Materials and methods

### 1) Main reagents

Omaveloxolone was purchased from MCE Biotechnology Co. Ltd.; Solutal was purchased from Sigma-Aldrich (Shanghai) Trading Co., Ltd.; and physiological saline was purchased from Sinopharm Chemical Reagent Co., Ltd.

### 2) Animals

60 B6SJL-Tg(SOD1 G93A)-1Gur/J transgenic mice (model for amyotrophic lateral sclerosis, ALS), half male and half female, were purchased from Shanghai Model Organisms Center, Inc.

### 3) Experimental grouping and administration

SOD1 G93A mice were randomly divided into the model group, Omaveloxolone 1 mg/kg group, Omaveloxolone 3 mg/kg group, compound 26 0.99 mg/kg group and compound 28 1.06 mg/kg group, with 12 mice in each group. Concurrently, 12 C57BL/6J mice were set as the control group. Drugs were administered intraperitoneally. The control group and the model group were given an equal volume of vehicle (1% DMSO + 4% solutal + 95% physiological saline). Each group was administered once daily for 10 consecutive weeks.

### 4) Indicator detection

### Rotarod test

The rotarod test is a classical behavioral test used to evaluate motor coordination in mice. Rotarod test (Panlab rotarod apparatus, purchased from Harvard Bioscience, USA) was performed twice each week. The rotating speed range of the rotarod was from 4 r/min to 40 r/min. Experimental procedures were as follows: 1. Before the formal experiment, mice underwent habituation training with the rotation speed set at 12 r/min, for 5 min each time, twice daily for 3 consecutive days; 2. Formal experiment: three consecutive tests (3 min/each test, with a 30 min interval between tests); 3. The time each mouse stayed on the rotarod was recorded for each test, and the longest time on the rotarod among the three tests was taken as the fall latency.

### Onset time

The rotarod test was used to determine the onset time of disease in mice. The date when a mouse first fell off the rotarod within 3 minutes was recorded as the onset date.

### Hanging cage test

The hanging cage test was used to assess the grip strength and endurance of the mouse's limbs. Hanging cage test was performed twice each week. Each mouse was placed on the center of a wire mesh, which was gently shaken to make the mouse grip tightly. The wire mesh was then slowly inverted to a horizontal position, and the time the mouse hung onto the wire mesh was recorded. Each mouse was measured three times with a 30-minute interval between tests, and the longest time thereof was taken as the fall latency.

### 2. Experimental results

### 1) Effects of compounds 26 and 31 on the onset time in SOD1 G93A mice

As shown in FIG. 5, Omaveloxolone 1 mg/kg and 3 mg/kg both significantly delayed the onset time in SOD1 G93A mice, but there was no significant difference between the two groups, indicating that Omaveloxolone 1 mg/kg reached the maximum effect. At the same molar dose as Omaveloxolone 1 mg/kg, compound 26 (0.99 mg/kg) and compound 31 (1.06 mg/kg) both significantly delayed the onset time in SOD1 G93A mice, and the effect was superior to Omaveloxolone 3 mg/kg, with statistically significant differences.

### 2) Effects of compounds 26 and 31 on motor coordination in SOD1 G93A mice

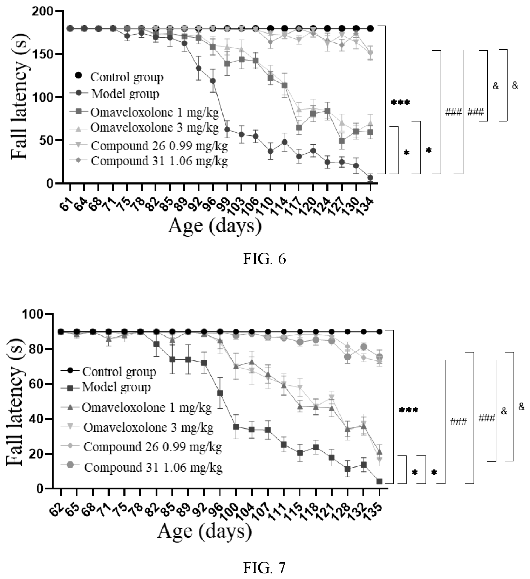

As shown in FIG. 6, Omaveloxolone 1 mg/kg and 3 mg/kg both significantly improved motor coordination in SOD1 G93A mice, as evidenced by a significant increase in fall latency. However, there was no significant difference between the two groups, indicating that Omaveloxolone 1 mg/kg reached the maximum effect. At the same molar dose as Omaveloxolone 1 mg/kg, compound 26 (0.99 mg/kg) and compound 31 (1.06 mg/kg) both significantly increased the fall latency in mice, and the effect was superior to Omaveloxolone 3 mg/kg, with statistically significant differences.

### 3) Effects of compounds 26 and 31 on muscle endurance in SOD1 G93A mice

As shown in FIG. 7, Omaveloxolone 1 mg/kg and 3 mg/kg both significantly improved muscle endurance in SOD1 G93A mice, as evidenced by a significant increase in fall latency. However, there was no significant difference between the two groups, indicating that Omaveloxolone 1 mg/kg reached the maximum effect. At the same molar dose as Omaveloxolone 1 mg/kg, compound 26 (0.99 mg/kg) and compound 31 (1.06 mg/kg) both significantly increased the fall latency in mice, and the effect was superior to Omaveloxolone 3 mg/kg, with statistically significant differences.

### 3. Experimental conclusion

Compounds 26 and 31 improves the neurobehavioral functions in amyotrophic lateral sclerosis model mice, and its effect is superior to that of Omaveloxolone.

In summary, the compounds involved in the present application, as novel NRF2 activators, exhibit excellent Nrf2 agonist activity. These compounds exert their antioxidant effects through novel mechanisms such as scavenging DPPH free radicals or inhibiting the formation of lipid peroxides MDA. Furthermore, *in vivo* experiments of the present application also demonstrated that these compounds provided by the present application are more effective in preventing or treating related diseases, includingmitochondrial encephalomyopathy, autism spectrum disorder, Rett syndrome, Friedreich ataxia, stroke, hemorrhagic stroke, ischemic stroke, multiple sclerosis, amyotrophic lateral sclerosis (ALS), schizophrenia, schizophrenia with cognitive impairment, Parkinson's disease, Parkinson's cognitive impairment, Alzheimer's disease, vascular dementia, epilepsy, Huntington's chorea, heart failure, myocardial infarction, renal failure, and renal ischemia. In particular, some compounds show significant therapeutic and/or preventative effects against stroke, multiple sclerosis, and amyotrophic lateral sclerosis.

The preferred embodiments of the present application have been described in detail above. However, the present application is not limited to the specific details in the above embodiments. Within the scope of the technical concept of the present application, various equivalent transformations may be made to the technical solutions of the present application, and these equivalent transformations all fall within the protection scope of the present application.

## Claims

1. A compound of formula (I) or pharmaceutically acceptable salt thereof, as shown below: 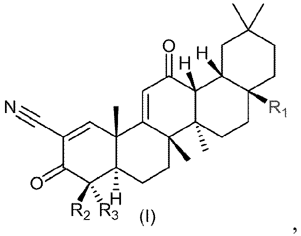 wherein:
R₁ is independently selected from the group consisting of: -C(=O)-alkyl, -C(=O)-substituted alkyl, -C(=O)-alkenyl, -C(=O)-substituted alkenyl, -C(=O)-alkynyl, -C(=O)-substituted alkynyl, - C(=O)-heteroaryl, -C(=O)-heteroaryl-R₁', -C(=O)-heteroaryldiyl-R₁'-C(=O)-NH-OH, -C(=O)-N(OH)-alkyl, -C(=O)-N(OH)-substituted alkyl, -C(=O)-N(OH)-alkenyl, -C(=O)-N(OH)-substituted alkenyl, -C(=O)-N(OH)-alkynyl, -C(=O)-N(OH)-substituted alkynyl, -C(=O)-N(OH)-aryl, -C(=O)-N(OH)-aryldiyl-R₁', -C(=O)-N(OH)-heteroaryl, -C(=O)-N(OH)-heteroaryldiyl-R₁', - C(=O)-NH-aryl, -C(=O)-NH-aryldiyl-R₁', -C(=O)-NH-heteroaryl, -C(=O)-NH-heteroaryldiyl-R₁', -C(=O)-O-aryl, -C(=O)-O-aryldiyl-R₁', -C(=O)-O-heteroaryl, -C(=O)-O-heteroaryldiyl-R₁', - C(=O)-CH₂-heteroaryl, -C(=O)-CH₂-heteroaryldiyl-R₁', -C(=O)-CR₂'R₃'-heteroaryl, -C(=O)-CR₂'R₃'-heteroaryldiyl-R₁', -C(=O)-L-amino acid-NH-heteroaryl, and -C(=O)-L-amino acid-NH-heteroaryldiyl-R₁'; and
R₂ is hydrogen or methyl, and R₃ is hydrogen or methyl.

2. The compound according to claim 1, wherein the compound is: 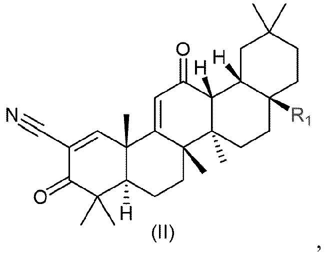 wherein:
R₁ is independently selected from the group consisting of: -C(=O)-alkyl, -C(=O)-substituted alkyl, -C(=O)-alkenyl, -C(=O)-substituted alkenyl, -C(=O)-alkynyl, -C(=O)-substituted alkynyl, - C(=O)-heteroaryl, -C(=O)-heteroaryl-R₁', -C(=O)-heteroaryldiyl-R₁'-C(=O)-NH-OH, -C(=O)-N(OH)-alkyl, -C(=O)-N(OH)-substituted alkyl, -C(=O)-N(OH)-alkenyl, -C(=O)-N(OH)-substituted alkenyl, -C(=O)-N(OH)-alkynyl, -C(=O)-N(OH)-substituted alkynyl, -C(=O)-N(OH)-aryl, -C(=O)-N(OH)-aryldiyl-R₁', -C(=O)-N(OH)-heteroaryl, -C(=O)-N(OH)-heteroaryldiyl-R₁', - C(=O)-NH-aryl, -C(=O)-NH-aryldiyl-R₁', -C(=O)-NH-heteroaryl, -C(=O)-NH-heteroaryldiyl-R₁', -C(=O)-O-aryl, -C(=O)-O-aryldiyl-R₁', -C(=O)-O-heteroaryl, -C(=O)-O-heteroaryldiyl-R₁', - C(=O)-CH₂-heteroaryl, -C(=O)-CH₂-heteroaryldiyl-R₁', -C(=O)-CR₂'R₃'-heteroaryl, -C(=O)-CR₂'R₃'-heteroaryldiyl-R₁', -C(=O)-L-amino acid-NH-heteroaryl, and -C(=O)-L-amino acid-NH-heteroaryldiyl-R₁'; and
R₂ is methyl; R₃ is methyl.

3. The compound according to claim 1 or 2, wherein in aromatic compounds, R₁' is independently selected from the group consisting of:
-Cl, -F, -Br, -OH, isopropyl, linear/branched alkyl (C≤6), linear/branched alkyl (C≤6) substituted with 1-5 halogens, -OH, linear/branched alkyl (C≤6) substituted with 1-5 -OHs, linear/branched alkenyl (C≤6), linear/branched alkenyl (C≤6) substituted with 1-5 halogens, linear/branched alkenyl (C≤6) substituted with 1-5 -OHs, linear/branched alkynyl (C≤6), linear/branched alkynyl (C≤6) substituted with 1-5 halogens, linear/branched alkynyl (C≤6) substituted with 1-5 -OHs,

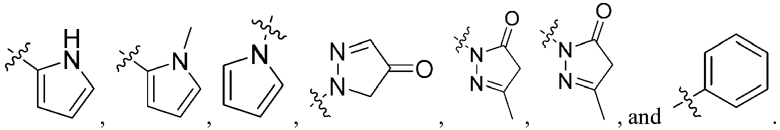

4. The compound according to claim 3, wherein the aryl is selected from the group consisting of: 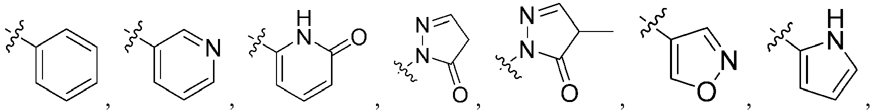 and

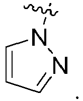

5. The compound according to claim 3, wherein substituted alkyls, substituted alkenyls, substituted alkynyls, alkyls, alkenyls, and alkynyls have a C chain length of ≤6, and are each independently linear, branched, or cyclic.

6. The compound according to claim 1, wherein the compound is as follows:

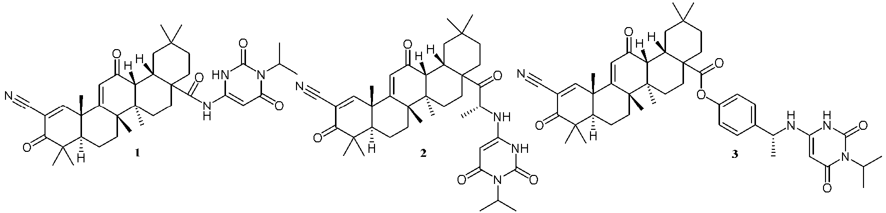

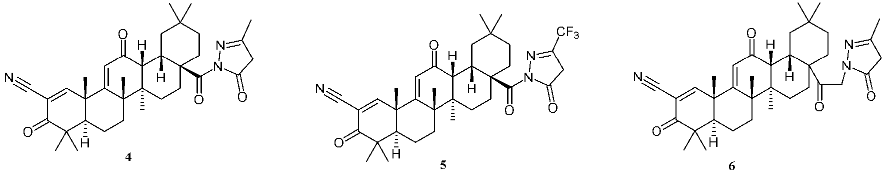

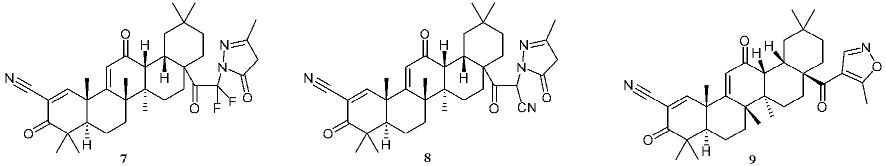

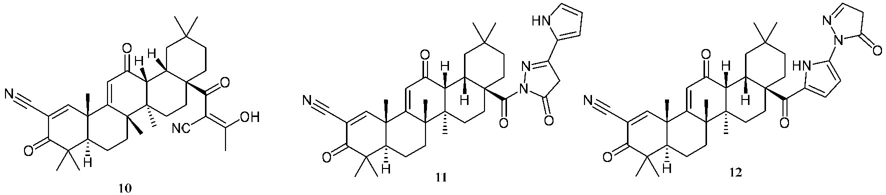

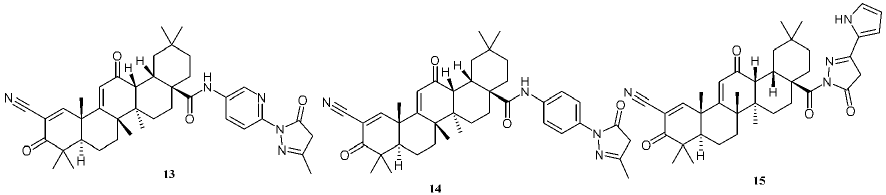

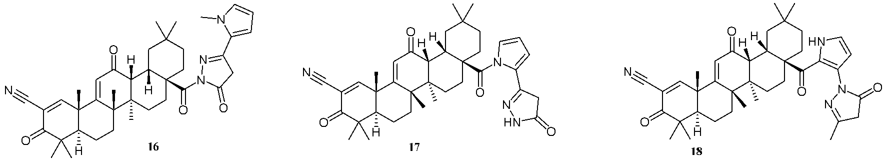

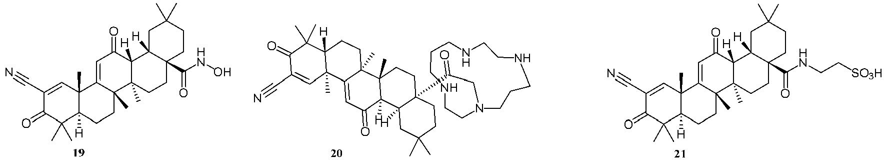

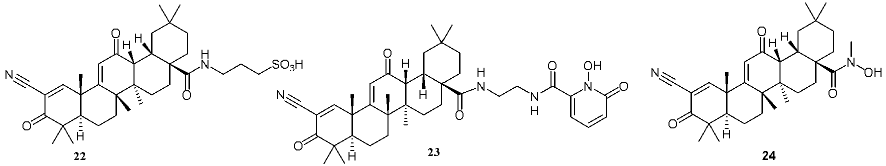

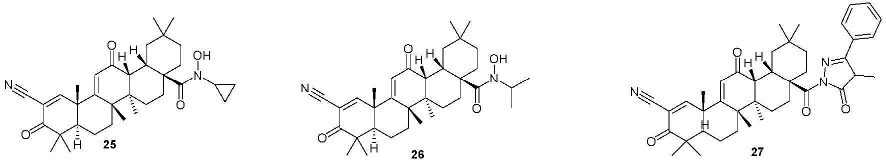

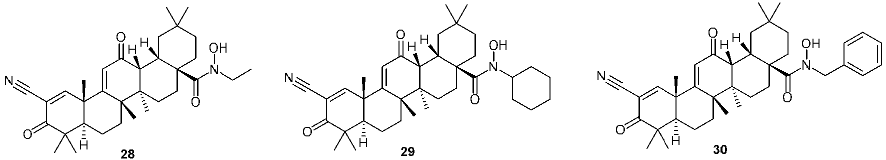

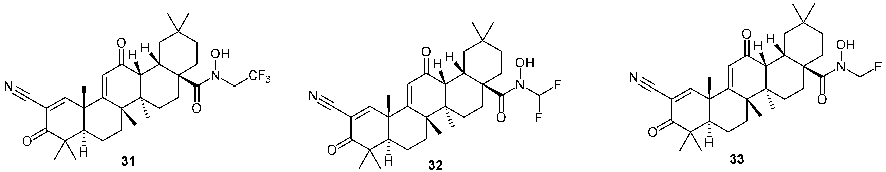

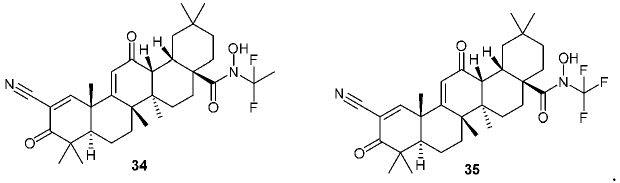

7. Use of the compound according to claim 6 in preparation of an NRF2 activator.

8. Use of the compound according to claim 6 in preparation of a medicament for treating and/or preventing a disease in a patient, wherein the prepared medicament is used to prevent or treat the disease in the patient, comprising mitochondrial encephalomyopathy, autism spectrum disorder, Rett syndrome, Friedreich ataxia, stroke, hemorrhagic stroke, ischemic stroke, multiple sclerosis, amyotrophic lateral sclerosis (ALS), schizophrenia, schizophrenia with cognitive impairment, Parkinson's disease, Parkinson's cognitive impairment, Alzheimer's disease, vascular dementia, epilepsy, Huntington's chorea, heart failure, myocardial infarction, renal failure, and renal ischemia.

9. The use according to claim 8, wherein the compound is used in preparation of a medicament for preventing or treating stroke, multiple sclerosis, and amyotrophic lateral sclerosis.
